(19) Europäisches Patentamt / European Patent Office / Office européen des brevets

(11) EP 3 423 569 B1

(12) # EUROPEAN PATENT SPECIFICATION

(45) Date of publication and mention of the grant of the patent:
**21.02.2024 Bulletin 2024/08**

(21) Application number: **17709749.0**

(22) Date of filing: **28.02.2017**

(51) International Patent Classification (IPC):
***C12N 5/077** (2010.01)* ***C12N 5/00** (2006.01)*
***C12M 3/02** (2006.01)* ***C12M 1/26** (2006.01)*

(52) Cooperative Patent Classification (CPC):
**C12N 5/0062; C12M 25/14; C12M 33/00;**
C12N 2531/00; C12N 2533/52; C12N 2533/54; C12N 2533/76; C12N 2535/10

(86) International application number:
**PCT/GB2017/050541**

(87) International publication number:
**WO 2017/149296 (08.09.2017 Gazette 2017/36)**

(54) **3D PRINTING OF A CELLULARISED SCAFFOLD**

3D-DRUCKEN EINES ZELLULÄRISIERTEN GERÜSTS

IMPRESSION 3D D'UN ÉCHAFAUDAGE OCCUPÉ PAR DES CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.03.2016 GB 201603564**
**01.03.2016 GB 201603560**

(43) Date of publication of application:
**09.01.2019 Bulletin 2019/02**

(73) Proprietor: **Oxford University Innovation Limited**
**Oxford, Oxfordshire OX2 0JB (GB)**

(72) Inventors:
- **BAYLEY, Hagan**
  **Oxford**
  **Oxfordshire OX1 3TA (GB)**
- **OLOF, Sam**
  **Oxford**
  **Oxfordshire OX1 3TA (GB)**
- **GRAHAM, Alexander D**
  **Oxford**
  **Oxfordshire OX1 3TA (GB)**
- **BURKE, Madeline**
  **Sutton**
  **Surrey SM1 2BE (GB)**
- **ARMSTRONG, James**
  **Swindon**
  **Wiltshire SN1 3AG (GB)**
- **PERRIMAN, Adam**
  **Bristol BS1 6HL (GB)**
- **NICHOLSON, James**
  **Oxford**
  **Oxfordshire OX1 3QU (GB)**
- **SZELE, Francis**
  **Oxford**
  **Oxfordshire OX1 3PT (GB)**

(74) Representative: **J A Kemp LLP**
**80 Turnmill Street**
**London EC1M 5QU (GB)**

(56) References cited:
**WO-A1-2011/151832** **WO-A2-2014/087175**
**US-A1- 2013 202 802** **US-A1- 2014 356 289**
**US-A1- 2015 248 949**

- **DANIELA F DUARTE CAMPOS ET AL: "Three-dimensional printing of stem cell-laden hydrogels submerged in a hydrophobic high-density fluid", BIOFABRICATION, vol. 5, no. 1, 1 March 2013 (2013-03-01), - 1 March 2013 (2013-03-01), page 015003, XP055060280, ISSN: 1758-5082, DOI: 10.1088/1758-5082/5/1/015003**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DANIELA F. DUARTE CAMPOS ET AL: "The Stiffness and Structure of Three-Dimensional Printed Hydrogels Direct the Differentiation of Mesenchymal Stromal Cells Toward Adipogenic and Osteogenic Lineages", TISSUE ENGINEERING PART A, vol. 21, no. 3-4, 1 February 2015 (2015-02-01), - 1 February 2015 (2015-02-01), pages 740-756, XP055370230, US ISSN: 1937-3341, DOI: 10.1089/ten.tea.2014.0231
- GUDAPATI HEMANTH ET AL: "A comprehensive review on droplet-based bioprinting: Past, present and future", BIOMATERIALS, vol. 102, 7 June 2016 (2016-06-07), pages 20-42, XP029630710, ISSN: 0142-9612, DOI: 10.1016/J.BIOMATERIALS.2016.06.012

## Description

### FIELD OF THE INVENTION

The present invention relates to a process for producing a droplet assembly as defined in claim 1, and to a droplet assembly obtainable by such a process. The invention also relates to a droplet assembly comprising a plurality of droplets as defined in claim 9, and to a composition as defined in claim 14.

### BACKGROUND OF THE INVENTION

There is a demand to develop tissues with intrinsic properties which may be used in the field of regenerative medicine or as empirical models for drug screening. To develop a tissue, multiple cells of various types must be spatially arranged at a high density within a cytocompatible environment, such that they will proliferate and recapitulate the function of desired tissues. Bioprinting has offered a way of patterning heterogeneous cell types at low to high resolution with low to high cell densities depending on the method. However, high resolution patterning is usually done at the expense of using low cell density materials.

Aqueous droplets formed in a lipid-in-oil solution will be spontaneously coated in a lipid monolayer. When monolayer-coated droplets are brought together they will cohere through a lipid bilayer (Syeda et al, J. Am. Chem. Soc. 130, 15543-8 (2008)). Multiple droplets can be cohered, connected through these droplet interface bilayers (DIBs), forming multicompartmentalised networks that can be produced in 2D or 3D (Villar et al, Science 340, 48-52 (2013); Maglia et al, Nat. Nanotechnol. 4, 437-440 (2009); Elani et al, Lab Chip 12, 3514-20 (2012); Villar et al, Nat. Nanotechnol. 6, 803-8 (2011); Elani et al, Chem. Sci. 4, 3332 (2013)).

Multisomes are aqueous droplet networks suspended in a bulk aqueous phase (Onoe et al, Drug Discov. Today 20, 236-246 (2015)). They are able to interact with the bulk phase through use of specific lipid mixtures which can be ruptured (either when the pH is lowered or temperature is changed). These structures were made by suspending the lipid-in-oil solution on a metal hoop in a bulk aqueous phase, and then forming droplets in the oil. Hence, all multisome structures formed were of spheroid like or ellipsoid structure.

WO 2014/064459 describes hydrogel networks comprising hydrogel bodies having an outer layer of amphipathic molecules. WO 2014/087175 describes an apparatus for producing a droplet assembly.

There is a need to develop a method which allows 3D cellularised scaffolds to be produced in a manner which is reproducible and allows access to finely structured cell-laden scaffolds with improved voxel resolution and high cell density.

### SUMMARY OF THE INVENTION

The inventors have developed a liquid-in-liquid 3D bioprinting method. Surprisingly, this method enables multiple cell-types to be patterned into high-resolution, spatially-defined architectures (such as those shown in Figure 2). This process provides significant improvements over previous methods and allows the fabrication of high cell density droplet assemblies with high resolution features, whilst retaining cell biological activity. The droplet assembly produced by the process of the invention serves as a celluralised scaffold which may be cultured, leading to tissue-like materials. The process of the invention makes use of a unique bioink composed of agarose in culture medium with supplements. Some of the advantages of the process of the invention are as described below.

There are multiple methods of making tissues, with bioprinting being just one. The general advantages of bioprinting over other tissue fabrication technologies and specific advantages of the claimed bioprinting method over current bioprinters are discussed below.

The process of the invention has advantages over organoid and spheroid culture methods. Methods such as organogenesis in a dish (Lancaster et al, Science (80), 345, 1247125 1-9, 2014) and spheroid culture (Foty, R, J. Vis. Exp. 20, 4-7, 2011) rely only on a mix of cells redistributing and growing together naturally within a hydrogel or culture medium. The initial mix of cells either consists of a ratio of differentiated cell-types, or a single pluripotent cell-line which will differentiate into heterogeneous lineages. While these tissues show native properties, they can only grow to a size that is within the diffusion limit and the architecture may vary across a set of samples. Hence, there is little control over the tissue structure by the researcher. The bioprinting process of the invention offers spatial control of cell seeding within an environment, meaning the shape and architecture of the tissue will be better defined. The printed structures have the potential to be much larger than organoids.

The process of the invention also has advantages over cell-laden microfibres and cell sheets technology. Cell sheet technology (CST) (Haraguchi, Y, RSC Adv. 2, 2184, 2012) involves the stacking of cultured cell sheets without the presence of a culture hydrogel or supporting scaffold. CST structures can form tissues with intrinsic properties due to their retention of extracellular membranes (ECM) during construction. However, the process is slow and requires

parallel culture of each layer before stacking. There is also limited patterning control within a single sheet.

Cell laden microfibres (CLM) (Onoe, H, Drug Discov. Today 20, 236-246, 2015) are hydrogel fibres, laden with cells which are typically made by microfluidic devices. These fibres can be weaved together to form 3D structures. These structures have been shown to have functional tissue properties and have been implanted into diabetic mice to release insulin (Onoe, H, Nat. Mater. 12, 584-90, 2013). This technique however is restricted to 3D structures, which are weavable.

The bioprinting process of the invention offers higher resolution spatial control of cell placement within a scaffold and can form shapes or structures unattainable by the technologies described above. Hence tissues with complex architectures of heterogeneous cells may only be achievable by bioprinting.

In particular, the invention provides a process for producing a droplet assembly, which droplet assembly comprises a plurality of droplets, wherein each of said droplets comprises:

an aqueous medium comprising (i) a hydrogel compound, which hydrogel compound is a polysaccharide, and (ii) one or more stabilising agents, which one or more stabilizing agents are selected from compounds which comprise a dipeptide; and
one or more biological cells disposed in the aqueous medium,

which process comprises:
generating, in a bulk hydrophobic medium comprising one or more amphipathic compounds, a plurality of droplets, wherein each of said droplets comprises:

an aqueous medium comprising (i) a hydrogel compound, which hydrogel compound is a polysaccharide, and (ii) one or more stabilising agents, which one or more stabilizing agents are selected from compounds which comprise a dipeptide; and
one or more biological cells disposed in the aqueous medium.

The invention also provides a droplet assembly comprising a plurality of droplets, wherein each of said droplets comprises:

(i) an aqueous medium comprising (i) a hydrogel compound, which hydrogel compound is a polysaccharide, and (ii) one or more stabilising agents, which one or more stabilizing agents are selected from compounds which comprise a dipeptide;
(ii) one or more biological cells disposed in the aqueous medium; and
(iii) an outer layer of amphipathic molecules around the surface of the aqueous medium,

wherein at least one droplet in the droplet assembly contacts at least one other droplet in the droplet assembly forming a layer of amphipathic molecules as an interface between contacting droplets.

The invention further provides a composition comprising a bulk hydrophobic medium and, disposed in the bulk hydrophobic medium, a droplet assembly of the invention as defined above.

The invention further provides a droplet assembly obtainable by the process of the invention as defined above.

## BRIEF DESCRIPTION OF THE FIGURES

Figure 1 shows a schematic diagram, micrographs and a graph demonstrating the 3D printing of cell networks.

Figure 2 shows micrographs demonstrating high resolution patterning of two cell types using the 3D printing process.

Figure 3 shows the phase transfer and culture of printed networks containing HEK-293T cells.

Figure 4 shows micrographs demonstrating the development of printed HEK-293T cells within networks over 7 days.

Figure 5 shows the effect of bioink cell density on network cell density.

## DETAILED DESCRIPTION OF THE INVENTION

The invention provides a process for producing a droplet assembly, which droplet assembly comprises a plurality of droplets, wherein each of said droplets comprises: an aqueous medium comprising (i) a hydrogel compound, which

hydrogel compound is a polysaccharide, and (ii) one or more stabilising agents, which one or more stabilizing agents are selected from compounds which comprise a dipeptide; and one or more biological cells disposed in the aqueous medium, which process comprises: generating, in a bulk hydrophobic medium comprising one or more amphipathic compounds, a plurality of droplets, wherein each of said droplets comprises: an aqueous medium comprising (i) a hydrogel compound, which hydrogel compound is a polysaccharide, and (ii) one or more stabilising agents, which one or more stabilizing agents are selected from compounds which comprise a dipeptide; and one or more biological cells disposed in the aqueous medium.

The term "droplet", as used herein, typically refers to any bound volume of a material (which may for instance be a liquid or a gel). The volume of a droplet is typically less than 1.0 mL, for instance less than 0.1 mL. For instance, a bound volume of an aqueous medium having a volume of less than 500 nL is a droplet. When first generated, a droplet may be substantially spherical in character (for instance as shown in Figure 1d). However, once in contact with other droplets in the droplet assembly a droplet may adopt a range of shapes. Typically, a droplet has a sphericity (e.g. the ratio of the surface area of a sphere of the same volume as the droplet to the actual surface area of the droplet) of greater than or equal to 0.5, for instance greater than or equal to 0.6. Thus, the greatest external dimension of a droplet (e.g. length) is typically less than or equal to 2.0 times the smallest external dimension of a droplet (e.g. width). Generating a droplet typically comprises disposing a volume of the aqueous medium within the bulk hydrophobic medium. The term "droplet of a medium" is typically equivalent to the term "volume of a medium".

A droplet assembly is a collection of droplets, which is typically arranged in a three dimensional array (for instance as shown in Figure 1e). Typically in a droplet assembly, each droplet is in contact with at least one other droplet in the assembly. A "droplet assembly" may also be referred to as a "assembly of volumes [of the material comprised in the droplets]". A droplet assembly produced by the process of the invention may be of any size. For instance, the largest external dimension of a droplet assembly may be from 0.01 mm to 100.0 mm. The largest external dimension of the droplet assembly may, in some cases, be less than or equal to 10.0 mm, for instance less than or equal to 5.0 mm.

A hydrogel compound is a compound such that an aqueous solution of the hydrogel compound is capable of gelling to form a hydrogel. Typically an aqueous medium comprising a hydrogel compound will gel to form a hydrogel when the temperature of the aqueous medium is reduced below a certain temperature (which will be the gelling temperature of the hydrogel compound in that aqueous medium). An aqueous composition comprising a hydrogel compound is not typically itself a gel but rather a free flowing liquid. It is only once such an aqueous composition comprising the hydrogel compound has gelled that the aqueous composition is a hydrogel. A gel may be defined as a "nonfluid colloidal network or polymer network that is expanded throughout its whole volume by a fluid". A hydrogel may be defined as "a gel in which the swelling agent (i.e. the fluid) is water".

A droplet assembly may also be referred to as a "scaffold", particularly when the droplet assembly has been gelled. The droplet assembly produced by the process comprises an aqueous medium comprising a hydrogel compound and may be un-gelled (i.e. where the aqueous medium remains as a free-flowing liquid comprising a hydrogel compound) or gelled (i.e. where the aqueous medium comprising the hydrogel compound has gelled to form a hydrogel).

A bulk hydrophobic medium is any volume of a hydrophobic medium which has a volume larger than the volume of a droplet of the aqueous medium. Often the bulk hydrophobic medium has a volume of greater than or equal to 0.5 mL or greater than or equal to 1.0 mL. For instance, the volume of the bulk hydrophobic medium may be greater than or equal to 10.0 mL. A hydrophobic medium is typically a hydrophobic liquid, for instance a liquid that is not substantially miscible with water.

The hydrogel compound is a polysaccharide. Examples of hydrogel compounds include agarose, methylcellulose and hyaluronan. Preferably, the hydrogel compound is agarose. The hydrogel compound typically has a gelling temperature of less than 20°C. For instance, the hydrogel compound may have a gelling temperature of less than or equal to 10°C or less than or equal to 5.0°C. The gelling temperature may be as measured for an aqueous solution of the hydrogel compound with a concentration of 10 mg/mL.

The concentration of the hydrogel compound in the aqueous medium is typically from 0.01 mg/L to 500.0 mg/L. For instance, the concentration of the hydrogel compound in the aqueous medium may be from 0.1 mg/L to 100.0 mg/L, or from 0.5 mg/L to 30.0 mg/L.

An aqueous medium may be any liquid medium comprising water. Typically, the aqueous medium is a composition comprising greater than or equal to 80 wt% water or greater than or equal to 90 wt% by water. The aqueous medium comprising a hydrogel compound typically comprises the hydrogel compound dissolved in water. The aqueous medium may comprise other components as described herein.

The aqueous medium further comprises one or more stabilising agents. The stabilising agents are usually dissolved in the aqueous medium. A stabilising agent is an agent (e.g. a compound) which increases the stability of the droplet assembly and/or improves the distribution of cells in the droplets in the droplet assembly.

The one or more stabilising agents are selected from compounds comprising a dipeptide. Preferably, the one or more stabilising agents are selected from dipeptides protected with an FMOC group. The FMOC group is the fluorenylmethyloxycarbonyl group. Thus, a dipeptide protected with an FMOC group is a compound of formula FMOC-X-X

where each X is independently an amino acid and each dash is a peptide bond. This may be represented by the formula below, wherein R' and R" are the variable groups in the amino acids.

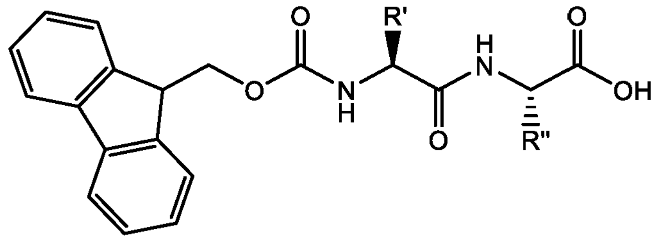

For instance, R' and R" may be independently selected from H, $CH_3$, $CH(CH_3)_2$, $CH_2CH(CH_3)_2$, $CH(CH_3)(CH_2CH_3)$ and $CH_2Ph$. Preferably, R' and R" are independently selected from H, $CH_3$, $CH(CH_3)(CH_2CH_3)$ and $CH_2Ph$. More preferably, the one or more stabilising agents are FMOC-isoleucine-glycine (FMOC-IG) and/or FMOC-phenylalanine-phenylalanine (FMOC-FF).

The concentration of the stabilising agent in the aqueous medium is typically from 0.01 to 100.0 mmol/L. For instance, the concentration of the one or more stabilizing agents may be from 0.1 to 10.0 mmol/L or from 0.5 to 2.0 mmol/L.

Typically, the aqueous medium further comprises an extracellular membrane protein (ECM). Examples of ECMs include collagen, elastin, fibronectin and laminin. The extracellular membrane protein preferably comprises collagen, fibronectin or laminin. The collagen may be any one of collagen types from I to XIV and is typically collagen Type I.

The concentration of the ECM in the aqueous medium is typically from 0.001 mg/L to 100.0 mg/L. For instance, the concentration of the ECM in the aqueous medium may be from 0.01 mg/L to 10.0 mg/L, or from 0.1 mg/L to 1.0 mg/L.

The aqueous medium often further comprises a culture medium. A culture medium is any aqueous medium suitable for culturing biological cells and culture media are well known to the skilled person. The culture medium is typically an aqueous solution of one or more amino acids (for instance glutamine or a source thereof), one or more salts (for instance sodium chloride or sodium pyruvate), glucose, and one or more vitamins (for instance vitamins A, B, C or D). The culture medium may further comprise one or more antibiotics. Examples of antibiotics include penicillin and streptomycin.

In some embodiments the aqueous medium comprises: (a) a hydrogel at a concentration of from 0.5 to 30.0 mg/mL; (b) a culture medium at a concentration of from 60.0 to 90.0 volume%; and (c) a compound comprising a dipeptide at a concentration of from 0.1 to 10.0 mmol/L. The aqueous medium may optionally further comprise (d) an extracellular membrane protein at a concentration of from 0.001 to 1.0 mg/mL. The osmolarity of the culture medium at pH 7.0 is typically from 200 to 400 mOsm, for instance from 300 to 350 mOsm.

The term "biological cell", as used herein, is well known and refers to a cell comprising a cytoplasm (typically comprising organelles such as a nucleus or a ribosome) enclosed within a membrane. The biological cells used in the process of the invention may be prokaryotic or eukaryotic. The biological cells are typically eukaryotic. The biological cells may be naturally occurring or genetically (or otherwise) modified. Often, the biological cells are mammalian cells derived from mammalian tissue, for instance mouse, rat, sheep or human tissue. For instance, the biological cells may be derived from primate tissue such as human or chimpanzee tissue.

In some embodiments, the one or more biological cells are selected from two or more different types of biological cells. Figure 2 shows examples of droplet assemblies containing two or more different types of biological cells.

A type of a biological cell refers to the cell type of a biological cell taken from a particular species. For instance, typical examples of mammalian biological cell types include human embryonic kidney (HEK) cells, osteoblast cells, chrondrocyte cells and mesenchymal stem cells.

Typically, if two or more cell types are present, one or more of the droplets each comprises two or more biological cells disposed in the aqueous medium, and the two or more biological cells are selected from two or more different types of biological cells. Thus, individual droplets may comprise two or more types of cell. Alternatively, different droplets may comprise different cell types allowing structural features containing different cell types to be incorporated into the droplet assembly. Thus, in some cases, the droplet assembly may comprise a first plurality of droplets, each of which droplets comprises one or more of a first type of biological cells disposed in the aqueous medium, and a second plurality of droplets, each of which droplets comprises one or more of a second type of biological cells disposed in the aqueous medium.

The one or more biological cells are typically mammalian cells.

Typically, the one or more biological cells are disposed in the aqueous medium at a concentration of from $10^5$ to $10^9$ cells per mL of aqueous medium. For instance, the concentration of the biological cells disposed in the aqueous medium may be from $10^5$ to $10^8$ cells per mL or from $10^6$ to $10^9$ cells per mL. Preferably the concentration of biological cells in the aqueous medium is greater than or equal to $10^7$ cells per mL. Droplet networks comprising different concentrations of biological cells are shown in Figure 5.

The bulk hydrophobic medium may be any hydrophobic medium. For instance, the bulk hydrophobic medium may comprise an organic compound. Typically, the bulk hydrophobic medium comprises a hydrocarbon compound. Often, the bulk hydrophobic medium comprises a hydrocarbon compound and/or a silicone oil.

A hydrocarbon compound is a compound comprising only carbon and hydrogen atoms. Examples of hydrogen compounds include $C_4$ to $C_{20}$ alkanes (for instance straight chain alkanes having from 6 to 18 carbon atoms) and $C_5$ to $C_{10}$ cycloalkanes (for instance cyclopentane or cyclohexane). Preferably, the hydrocarbon compound is a $C_8$ to $C_{16}$ alkane, for instance octane, nonane, decane, undecane or dodecane. Preferably, the hydrocarbon compound is undecane.

A silicone oil is an oil comprising a polymeric compound which comprises one or more siloxane groups. For instance, a silicone oil is typically a polymerized siloxane with organic side chains. For instance the silicone oil may comprise polydimethylsiloxane, polyethylmethylsiloxane or polydiethylsiloxane.

The bulk hydrophobic medium may for instance comprise a mixture of a hydrocarbon and a silicone oil in a ratio (hydrocarbon):(silicone oil) of from 50:50 to 80:20 by volume. Preferably, the ratio is from 60:40 to 70:30 by volume. The bulk hydrophobic medium may be a mixture of undecane and silicone oil in a ratio of from 60:40 to 70:30 by volume.

The bulk hydrophobic medium comprises one or more amphipathic compounds. An amphipathic compound is a compound comprising both hydrophilic groups and lipophilic groups (e.g. hydrophobic groups). Amphipathic molecules are typically able to form bilayers and micelles. Amphipathic molecules are well known to the skilled person.

The one or more amphipathic compounds may be selected from lipids. Examples of lipids include triglycerides, fatty acids and phospholipids. Typically, the one or more amphipathic compounds are selected from phospholipids. A phospholipid is compound comprising a glycerol molecule substituted with a phosphate group and one or more fatty acid groups. The one or more amphipathic compounds are preferably selected from phosphocholine lipids.

Examples of amphipathic molecules useful in the process of the invention include diphytanoylphosphatidylcholine, diphytanoylphosphatidylethanolamine, 1,2-didecanoyl-sn-glycero-3-phosphocholine, 1,2-dierucoyl-sn-glycero-3-phosphate, 1,2-dierucoyl-sn-glycero-3-phosphocholine, 1,2-dierucoyl-sn-glycero-3-phosphoethanolamine, 1,2-dilinoleoyl-sn-glycero-3-phosphocholine, 1,2-dilauroyl-sn-glycero-3-phosphate, 1,2-dilauroyl-sn-glycero-3-phosphocholine, 1,2-dilauroyl-sn-glycero-3-phosphoethanolamine, 1,2-dilauroyl-sn-glycero-3-phosphoserine, 1,2-dimyristoyl-sn-glycero-3-phosphate, 1,2-dimyristoyl-sn-glycero-3-phosphocholine, 1,2-dimyristoyl-sn-glycero-3-phosphoethanolamine, 1,2-dimyristoyl-sn-glycero-3-phosphoserine, 1,2-dioleoyl-sn-glycero-3-phosphate, 1,2-dioleoyl-sn-glycero-3-phosphocholine, 1,2-dioleoyl-sn-glycero-3-phosphoethanolamine, 1,2-dioleoyl-sn-glycero-3-phosphoserine, 1,2-dipalmitoyl-sn-glycero-3-phosphate, 1,2-dipalmitoyl-sn-glycero-3 - phosphocholine, 1,2-dipalmitoyl-sn-glycero-3-phosphoethanolamine, 1,2-dipalmitoyl-sn-glycero-3-phosphoserine, 1,2-distearoyl-sn-glycero-3-phosphate, 1,2-distearoyl-sn-glycero-3-phosphocholine, 1,2-distearoyl-sn-glycero-3-phosphoethanolamine, 1,2-distearoyl-sn-glycero-3-phosphoserine, egg-PC, hydrogenated egg PC, hydrogenated soy PC, 1-myristoyl-sn-glycero-3-phosphocholine, 1-palmitoyl-sn-glycero-3-phosphocholine, 1-stearoyl-sn-glycero-3-phosphocholine, 1-myristoyl-2-palmitoyl-sn-glycero 3-phosphocholine, 1-myristoyl-2-stearoyl-sn-glycero-3-phosphocholine, 1-palmitoyl-2-myristoyl-sn-glycero-3-phosphocholine, 1-palmitoyl-2-oleoyl-sn-glycero-3-phosphocholine, 1-palmitoyl-2-oleoyl-sn-glycero-3-phosphoethanolamine, 1-palmitoyl-2-stearoyl-sn-glycero-3-phosphocholine, 1-stearoyl-2-myristoyl-sn-glycero-3-phosphocholine, 1-stearoyl-2-oleoyl-sn-glycero-3-phosphocholine and 1-stearoyl-2-palmitoyl-sn-glycero-3-phosphocholine.

The total concentration of the one or more amphipathic compounds in the bulk hydrophobic medium may be from 0.01 mM to 100 mM. Typically, the concentration is from 0.1 mM to 10 mM, for instance from 0.5 mM to 5.0 mM. For example, the bulk hydrophobic medium may comprise diphytanoylphosphatidylcholine or diphytanoylphosphatidylethanolamine at a concentration of from 0.5 mM to 5.0 mM.

Generating the plurality of droplets typically comprises: (i) a plurality of dispensing steps, each of which dispensing steps comprises injecting into the bulk hydrophobic medium a volume of the aqueous medium with the one or more biological cells disposed therein.

The volume of the aqueous medium with the one or more biological cells disposed therein is typically from 0.001 to 1000 nL. For instance, the volume of the aqueous medium may be from 0.1 nL to 500 nL or from 1.0 nL to 300 nL. Often, the volume of the aqueous medium injected is from 50 nL to 250 nL.

Generating the plurality of droplets typically comprises 10 or more of the dispensing steps, for instance 100 or more of the dispensing steps. In some cases there may be a large number of dispensing steps, for instance 10,000 or more of the dispensing steps.

Typically, generating the plurality of droplets comprises using an apparatus for generating droplets, which apparatus comprises (a) at least one droplet generator; (b) a container which is movable relative to the at least one droplet generator; and (c) a control unit which is adapted to control the dispensing of droplets from the at least one droplet generator and to control the movement of the container relative to the at least one droplet generator. An example of an outlet of a droplet generator is partly shown in Figures 1a and 1d.

The (or each) droplet generator may for instance comprise: a chamber for holding the aqueous medium with

the one or more biological cells disposed therein; an outlet; and a component for displacing through said outlet a volume of the aqueous medium with the one or more biological cells disposed therein and thereby dispensing said volume as a droplet.

Examples of droplet generators and apparatuses for generating droplets suitable for use in the process of the invention may are described in WO 2014/087175, the entirety of which is incorporated by reference. The component for displacing through said outlet a volume of the aqueous medium with the one or more biological cells disposed therein is often a piezoelectric transducer. The container which is movable relative to the at least one droplet generator usually comprises the bulk hydrophobic medium.

Typically, the apparatus comprises: at least one droplet generator; a container which is moveable relative to the at least one droplet generator; and a control unit, which control unit is adapted to control the dispensing of droplets from the at least one droplet generator and the movement of the container relative to the at least one droplet generator, wherein the apparatus is adapted to produce a droplet assembly which comprises a plurality of droplets, wherein each of said droplets comprises (i) a droplet medium, and (ii) an outer layer of amphipathic molecules around the surface of the droplet medium, wherein the droplet medium is an aqueous medium, and wherein at least one of said droplets contacts another of said droplets to form a layer of said amphipathic molecules as an interface between the contacting droplets.

The apparatus may be adapted to produce a said droplet assembly wherein each of said droplets contacts another of said droplets to form a layer of said amphipathic molecules as an interface between the contacting droplets. The control unit is typically adapted to coordinate (a) the movement of the container relative to the or each droplet generator and (b) the dispensing of the droplets, to create said droplet assembly. The control unit may comprise a computer or dedicated electronic hardware. The apparatus often further comprises a micromanipulator for moving the container, and wherein the control unit is adapted to control movement of the container using the micromanipulator. The apparatus may further comprise a micromanipulator for moving the or each droplet generator and, when the apparatus comprises more than one said droplet generator, for coordinating the relative displacement of the droplet generators. Each droplet generator typically comprises: a chamber for holding a droplet medium, which is an aqueous medium; an outlet; and a component for displacing a volume of said droplet medium through said outlet and thereby dispensing said volume as a droplet. The outlet typically has a diameter of less than 200 $\mu$m. In some cases, the or each droplet generator is adapted to dispense droplets having a diameter of equal to or less than 1 mm, for instance from 10 $\mu$m to 200 $\mu$m. The or each droplet generator is typically adapted to dispense droplets having a volume of from 0.001 nL to 100 nL. The control unit is typically adapted to control the dispensing of droplets at a rate of from 0.01 to 10 $s^{-1}$. The apparatus in some cases may comprise a plurality of said droplet generators, for instance a first droplet generator comprises a first aqueous medium and wherein a second droplet generator comprises a second aqueous medium, wherein the first and second aqueous media are different (typically because they comprise different types of biological cells).

Typically, at least one of the droplets further comprises an outer layer of amphipathic molecules around the surface of the aqueous medium. A schematic example of droplets comprising an outer layer of amphipathic molecules is shown in Figure 1a. Such outer layers of amphipathic molecules are visible in Figure 1b. Typically, at least one of said droplets further comprises an outer layer of amphipathic molecules around the surface of the aqueous medium and contacts another one of said droplets which further comprises an outer layer of amphipathic molecules around the surface of the aqueous medium, and wherein a layer of amphipathic molecules is formed between the first and second droplets as an interface.

The droplet assembly often comprises a plurality of droplets arranged in a three-dimensional structure wherein each droplet in the three dimensional structure comprises an outer layer of amphipathic molecules around the surface of the aqueous medium and wherein each droplet in the three dimensional structure contacts at least one other droplet in the three dimensional structure forming a layer of amphipathic molecules as an interface between contacting droplets.

The three dimensional structure may comprise different layers of droplets, wherein each layer of droplets comprises a type of biological cell disposed therein and the types of biological cell is different in each of the different layers.

The layer of amphipathic molecules formed between the first and second droplets as an interface is typically a bilayer of amphipathic molecules formed between the first and second droplets as an interface. The outer layer of amphipathic molecules around the surface of the aqueous medium in one or more of the droplets may in some cases further comprise one or more transmembrane proteins.

Only small molecules such as water and gases like oxygen can permeate the bilayers, meaning droplet contents remain isolated. If a protein pore such as alpha haemolysin is present however, small molecules that can pass through the pores are able to flow across such interfaces. As such, emergent rapid communication properties have been seen within droplet networks containing protein pores across a pathway of droplets. Networks have also shown cooperativity when they contain regions of differing salt concentration, as the droplets will osmotically balance through osmosis, causing local droplet volume changes that could deform the whole network's shape i.e. the network can fold into a new shape.

In some cases, the bulk hydrophobic medium further comprises a transmembrane protein.

Often, the process further comprises, before the step of generating a plurality of droplets, a step of preparing the aqueous medium with one or more biological cells disposed therein, which preparation step comprises providing an aqueous medium and suspending a plurality of biological cells in the aqueous medium.

The process may further comprise a gelling step, which gelling step comprises allowing the aqueous medium comprising the hydrogel compound to form a gel. Typically, the gelling step comprises cooling the droplet assembly to a temperature of less than or equal to 10.0°C and allowing the aqueous medium comprising the hydrogel compound to form a gel. Alternative gelling methods (for instance exposure to UV light) may be used.

The process may further comprise culturing the plurality of biological cells to produce a celluralised droplet assembly. Culturing the plurality of cells typically comprises allowing the density of the biological cells to increase. The droplet assembly may be transferred to a culture medium prior to culturing. Culturing of the biological cells can be seen in Figure 4 where in Figures 4a-c, bright-field confocal micrographs show the networks (droplet assemblies) becoming dark and opaque over 7 days as the biological cells proliferated.

Often, the process further comprises isolating the droplet assembly from the bulk hydrophobic medium. This may be done by physically removing the droplet assembly from the bulk hydrophobic medium or alternatively by removing the bulk hydrophobic medium (e.g. by allowing it to drain).

The invention also provides a droplet assembly comprising a plurality of droplets, wherein each of said droplets comprises: (i) an aqueous medium comprising (a) a hydrogel compound, which hydrogel compound is a polysaccharide, and (b) one or more stabilising agents, which one or more stabilizing agents are selected from compounds which comprise a dipeptide; (ii) one or more biological cells disposed in the aqueous medium; and (iii) an outer layer of amphipathic molecules around the surface of the aqueous medium, wherein at least one droplet in the droplet assembly contacts at least one other droplet in the droplet assembly forming a layer of amphipathic molecules as an interface between contacting droplets.

The components and features of the droplet assembly may be as described above for the process of the invention.

The hydrogel compound is a polysaccharide, preferably agarose. The aqueous medium further comprises one or more stabilising agents selected from compounds comprising a dipeptide, such as dipeptides protected with a FMOC group. The one or more stabilising agents are most preferably FMOC-isoleucine-glycine and/or FMOC-phenylalanine-phenylalanine. For instance, the aqueous medium may comprise FMOC-isoleucine-glycine and FMOC-phenylalanine-phenylalanine. The aqueous medium may in some cases further comprise an extracellular membrane protein, such as collagen, fibronectin or laminin.

In some embodiments, the droplet assembly comprises: a first plurality of droplets, each of which droplets comprises one or more of a first type of biological cells disposed in the aqueous medium, and a second plurality of droplets, each of which droplets comprises one or more of a first type of biological cells disposed in the aqueous medium.

In the droplet assembly of the invention, each of the plurality of droplets typically has a volume of from 0.001 to 100 nL, or as defined above.

The droplet assembly of the invention may have gelled such that it is a cell-laden scaffold. Thus, in some cases the aqueous medium comprising a hydrogel compound is in the form of a gel.

The invention also provides a composition comprising a bulk hydrophobic medium and, disposed in the bulk hydrophobic medium, a droplet assembly of the invention as defined herein.

The invention also provides a droplet assembly obtainable by a process of the invention as defined herein.

## EXAMPLE

### Summary

Cell-laden droplet assemblies (scaffolds) were produced by 3D patterning a cell-laden bioink as a high cell density and high resolution droplet network (as shown in Figure 1), which was subsequently gelled. The resulting scaffold was phase transferred into bulk culture medium (as shown schematically in Figure 3a). The steps involved are: (i) creating the scaffold solution; (ii) re-suspending harvested cells in the scaffold solution as a bioink; (iii) 3D droplet printing of the bioink into droplet networks; (iv) gelation and phase transfer of the cell-scaffold. The cell scaffolds can then be cultured over a week or longer forming dense microtissues which show biological activity (as shown in Figure 4).

Figure 1a shows a schematic of the bio-printing process. The droplet dispensing nozzle ejects cell-containing bioink droplets into a lipid-rich oil. The droplets are spatially positioned by the programmed movement of the oil container. The layers of droplets cohere through the formation of droplet interface bilayers. Figure 1b is a confocal fluorescence micrograph showing droplet interface bilayers (stained yellow) within a cell-free printed network (11 $\times$ 14 $\times$ 7 droplets). The bilayers were visualised by adding sulforhodamine-101 (~10 $\mu$M) to the print solution. (Scale bar = 100 $\mu$m). A histogram showing the average HEK-293T cell density in printed droplets under oil as a function of the cell density in the bioink is shown in Figure 1c. The cell density was calculated as the average number of cells per droplet ($n$ = 25)

divided by the average droplet volume. Error bars represent the compound error of droplet size variance and cell per droplet variance. A bright-field micrograph of a cell network printed as successive layers of 1 nL droplets (d = 130 μm) ejected from the glass nozzle (d = -150 μm) is shown in Figure 1d. A confocal fluorescence micrograph of a printed HEK-293T cell network (11 × 14 × 2 droplets) under oil is shown in Figure 1e. Live/dead cell staining was performed with calcein-AM (CAM) and propidium iodide (PI), respectively. Visible are approximately 700 cells with a viability of 85% (determined by manual cell counting) (scale bar = 150 μm). A high magnification, confocal fluorescence micrograph of a live/dead assay performed on a HEK-293T cell network (7 × 8 × 4 droplets) printed at 15 × $10^6$ cells $mL^{-1}$, is shown in Figure If with an average occupancy of 38 cells per droplet (scale bar = 75 μm).

The steps are briefly described below and further detail can be found in the experimental details section.

***Scaffold solution:***

The scaffold solution is made of a hydrogel in a bulk cell culture medium with additional cell specific supplements where required. The hydrogel used was an ultra low gelling temperature agarose, as bioinks composed of this could be printed as a sol (or pre-gel liquid) at room temperature and then gelled at 4°C within a refrigerator. Supplements used were FMOC-dipeptides (FMOC-IG and FMOC-FF), which acted as both a network stabilising agent (i.e. prevented coalescence) and increased droplet-droplet adhesion within a network. Droplet-droplet adhesion is visible in Figure 1b. Extracellular matrix proteins (ECM) were also used to supplement the bioink, and these were either collagen type I, fibronectin or laminin, as these offered adhesion sites for the cells. It was empirically noticed that these ECMs encourage cell growth within the scaffold. In the majority of experiments the ECM supplement used was collagen type I.

***Resuspension of harvested cells as bioink:***

Cells grown by standard 2D culture methods were harvested from culture flasks and centrifuged to give a pellet. This cell pellet was then re-suspended in the scaffold solution at a typical cell density of 15×$10^6$ cells / mL. Cells were re-suspended at this high density as it was found the cells would form multiple contacts with one another in the printed structure and would proliferate better than other observed lower cell density scaffolds. Mammalian cell lines used included: HEK-293T, HEK-293/YFP, HEK-293/CFP, ovine mesenchymal stem cells, ovine osteoblasts, mouse chondrocytes and mouse tertiary neurospheres.

***3D bioprinting of the droplet networks:***

A 3D droplet printer was used to print the bioink as cell-laden droplet networks. The printing proceeded by first lowering the bioink-loaded print nozzle into a bath of lipid-in-oil solution, and finding print parameters that reproducibly formed droplets. These conditions could then be used to automate printing of 3D droplet networks by successive layering of spatially assigned droplets (based on uploaded "printing maps"). The optimised printing oil used was a 65:35 *V:V* (vol:vol) mix of undecane : silicone oil containing 1.2 mM DPhPC. This oil was designed to allow ideal printing (optimised droplet sinking speed) and the DPhPC was used as it forms stable bilayers. It was observed for bioink printed networks that the structure almost never showed any droplet-droplet coalescences, and formed a tightly packed structure. Such tightly packed droplet networks are shown in Figures 1e and 1f.

The process of the invention allows high resolution patterning of two cell types. Figures 2a-d show confocal fluorescence micrographs of printed cell-laden droplet assemblies produced by the process of the invention. HEK-293 cells stained with Deep Red (DR) or Red CMPTX (RC) CellTracker™ dyes were false-coloured blue and yellow, respectively. Figure 2a shows a Y-shaped structure within a square network (8 × 9 × 4 droplets), with an average feature width of 180 μm (scale bar = 200 μ). Figure 2b shows a cross-section at a defined z-height showing a single population of RC stained HEK-293 cells visible within a printed network, which comprised two discrete layers: lower RC-stained HEK-293 cells, 3 droplets thick; upper DR-stained HEK-293 cells, 4 droplets thick (scale bar = 250 μm). Figure 2c shows a cruciform pattern of HEK-293 cells within a square network (10 × 12 × 5 droplets) (scale = 250 μm). Figure 2d shows a high magnification image of the patterned HEK-293 cells in Figure 2c (scale bar = 100 μm). Figures 2e-h show side-on 3D images of lamellar networks, comprising CellTracker™-stained HEK-293 cells. Lower RC-stained HEK-293 cell layer, 3 droplets thick; upper RC-stained HEK-293 cell layer, 3 or 4 droplets thick. Images at: day 0, Figure 2e, immediately after printing and Figure 2f, immediately after transfer to culture medium; Figure 2g, day 3 in culture and; Figure 2h, day 5 in culture. (Scale bar = 250 μm).

***Network gelation and gel coating (phase transfer preparation):***

The printed networks were gelled by cooling to 4°C for 20-25 min. The gelled cargo-laden networks showed no loss in pattern fidelity. The structure was then coated with a small volume of agarose hydrogel. First the print oil was

exchanged for silicone oil and then a droplet of agarose was coalesced with the printed structure. The oil exchange was to remove the lipid, allowing coalescence rather than droplet interface bilayer formation to occur. The coated structure was then gelled (4 °C for 20-25 min).

***Phase transfer of cell scaffold:***

The gelled and coated droplet assembly was then phase-transferred by passing through an oil-culture medium interface, in this case a two phase column of a 3:1 V:V mix of hexadecane and mineral oil above cell culture medium. The cell scaffold was micropipetted into the upper oil phase and passed through the interface by gravity. After phase transfer the oil was removed and the cell scaffold was stored in a cell incubator and cultured over a week. A schematic diagram of the gelling and phase transfer process is shown in Figure 3a.

Figure 3b shows a 3D image of live/dead stained HEK-293T cells printed as a square network (7 $\times$ 8 $\times$ 4 droplets) imaged immediately after printing under oil. The printed droplets had an average density of 29 $\times$ $10^6$ cells $mL^{-1}$ with a viability of 96%. (Scale bar = 200 $\mu$m). Figure 3c shows 3D image of live/dead stained printed HEK-293T cells imaged after gel encapsulation and transfer to culture medium (scale bar = 200 $\mu$m). A graph showing HEK-293T cell viability (including standard error of the mean) of five printed networks at day 0 after transfer to culture medium is shown in Figure 3d. Viabilities were determined using automated object counting values, which were used either unmodified or resolved with respect to average cell size to represent live and dead cell numbers. Figure 3e shows a 3D image of immunocytochemistry performed on a network in culture medium at day 7: cell nuclei were stained with DAPI; cytoplasm of live cells was stained with CAM; and mitotic marker phospho histone-3 ICC, PH-3 was used (scale bar = 200 $\mu$m).

Figure 4 shows the development of printed HEK-293T cells within networks over 7 days. Figures 4a-i show images of individual printed HEK-293T cell network over 7 days of cell culture. Networks were printed with a bioink containing 15 $\times$ $10^6$ cells $mL^{-1}$ and 15 $\mu$g $mL^{-1}$ type I collagen. Constructs were imaged on day 0 (left-hand column), day 3 (middle column) and day 7 (right-hand column). Figures 4a-c are bright-field confocal micrographs of networks, where the networks became dark and opaque over 7 days as the cells proliferated. Figures 4d-f show 3D images of live/dead stained cell networks, which indicate a high viability throughout the course of culture. Figures 4g-i show composite bright-field and fluorescent micrographs showing magnified sections of printed cell networks. The cells had proliferated from individual entities to dense cell clusters. i, Cell clusters on day 7 were up to 140 $\mu$m wide. Scale bars are: a-f, 250 $\mu$m; g-h, 100 $\mu$m and; i, 30 $\mu$m.

Figure 5 shows the effect of bioink cell density on network cell density. Figures 5a-d show composite bright-field and fluorescent confocal micrographs of HEK-293T cell networks, printed at different bioink cells densities: a, 1 $\times$ $10^6$; b, 5 $\times$ $10^6$; c, 10 $\times$ $10^6$ and; d, 15 $\times$ $10^6$ cells $mL^{-1}$. The networks were created with a bioink containing live/dead dyes and were imaged immediately after printing while still in bulk oil. Images show: a-c, the entire network or d, cells compartmentalised within droplets. Figure 5e shows the average cell number per droplet for networks a-d, calculated from manual counts, here the average droplet sizes were: a, 120; b, 135; c, 130 and; d, 135 $\mu$m diameter. Figure 5f shows the average cell densities of droplets of networks shown in a-d, calculated as the average cell occupancy of droplets divided by the average droplet volume. Error bars represent either, the standard deviations of the cell count (e) or the compound errors of the counts and droplet volumes (f). Scale bars are: a-c, 250 $\mu$m and; d 75 $\mu$m.

**Experimental details:**

***Scaffold solution:***

This section goes through the scaffold solutions (also referred to as bioinks) that were used to fabricate phase transferrable scaffolds. First the bioink handling and compositions are explained and then how to make scaffold supplements.

<u>Solution Preparation Overview</u>

All scaffold solutions 1-3 (SS1-3) (see Table 1) were produced in the same manner: i.e. the bulk phase of the scaffold was first prepared as a liquid or pre-gel solution, then scaffold supplements were added and finally the cells resuspended at the desired density.

To minimise bacterial infection, different steps were taken to sterilise the scaffold solution. However, each bioink production used aseptic techniques and bioink processing was possible was performed in a laminar flow biosafety cabinet. The resulting cell-laden bioinks were stored in a $CO_2$ incubator [Midi 40, *Thermoscientific*] at 37°C with 5% $CO_2$ prior to use. Bioinks were generally being printed within 30 min of production, but have been stored up to 4 h prior to use.

Agarose-Based Solutions (SS1-SS3)

All agarose-based scaffold solutions consisted of ultralow gelling point agarose with or without scaffold supplements. These solutions were prepared as a liquid and kept above the agarose gel melting temperature (i.e. ~50°C) until the addition of ECM protein or cells, at which point the solution was brought to 37°C. Prior to the addition of cells, the solutions were UV irradiated, each for 15 min at 365 nm wavelength beneath an UV LED [Eclipse - M365L2-C5, *Nikon*] which was controlled by a LED driver [LEDD1B, *Thorlabs*] set to half power. All agarose-based scaffold solutions were used on the day of creation and not stored for future use.

*SS1* & *SS2:* Agarose with FMOC-XX

The FMOC-dipeptide supplemented agarose solutions were prepared as an 8:1 *V: V* mix of 13-15 mg/mL agarose with 10 mM FMOC-XX respectively. The original agarose solution *(SS6)* was undiluted, whilst the later diluted agarose solution (SS7) also contained 10% *V*/*V* PBS. UV treatment was carried out prior to cell addition for scaffold solution 6 and prior to PBS addition for scaffold solution 7.

The final composition of *scaffold solution 1* was: 11.6-13.3 mg/mL agarose and 1.1 mM FMOC-XX, in 88.9% base medium and 11.1% ultrapure water.

The final composition of *scaffold solution 2* was: 10.4-12.0 mg/mL agarose and 1.0 mM FMOC-XX, in 80% base medium, 10% ultrapure water and 10% PBS.

*SS3:* Agarose with FMOC-XX and ECM supplements

These bioinks involved supplementing *scaffold solution 7* with ECM proteins (either collagen, fibronectin or laminin) at various concentrations. The preparation was the same as scaffold solution 7, except, to the UV-sterilised agarose with FMOC-XX solution, ECM protein in PBS was initially added at the desired concentration, followed by just PBS to give a 10% *V*/*V* PBS fraction. The scaffold solution at this stage was sonicated (5 min, 40 kHz) in a 2800 ultrasonic cleaner [*Branson*]. The ECM proteins were supplemented in the concentration ranges of: 3.5-300 μg/mL for collagen; and 0.5-20 μg/mL for fibronectin and laminin. A supplement of 15 μg/mL collagen became a standard for microtissue growth.

Hence the final composition for the standard *scaffold solution 3* was: 10.4-12.0 mg/mL agarose, 1.0 mM FMOC-XX and 15 μg/mL collagen, in 80% base medium, 10% ultrapure water and 10% PBS.

Scaffold Supplement Preparation

10 mM FMOC-IG & 10 mM FMOC FF solutions

10 mM solutions of FMOC-isoleucine-glycine (FMOC-IG) and FMOC-phenylalanine-phenylalanine (FMOC-FF), were prepared from powder aliquots stored at -20°C, and allowed to warm to room temperature. FMOC-IG (16 mg) and FMOC-FF (12 mg) were separately dissolved in, ultrapure water (1.5 mL) with 1 M NaOH (10-20 μL), and left to stir overnight. The partially solubilised FMOC solution was sonicated (20 min, 37°C, 40 kHz) in a Branson 2800 ultrasonic cleaner, pH corrected with 0.1 mM NaOH to either 8.50 (FMOC-IG) or 10.50 (FMOC-FF), and then diluted to 3 mL total volume in ultrapure water. FMOC-IG and FMOC-FF were used within a week and pH corrected if necessary before use.

10 mM FMOC-XX Solution

An FMOC-XX solution was an equal molar ratio solution of FMOC-IG and FMOC-FF. For a 10 mM FMOC-XX solution, 10 mM FMOC-FF and 10 mM FMOC-IG were mixed as a 1:1 *V: V* solution and then sonicated (40 kHz). FMOC-XX was made fresh on the day of use.

Agarose Solutions

Ultralow gelling point agarose [A5030] was used to make the 13-15 mg/mL agarose solution. Typically agarose powder and base medium were warmed in a water bath (65°C). Warmed agarose powder (typically ~12 mg) was dissolved in warmed base medium (typically ~0.8 mL). To aid solvation, the solution was vortex mixed and, optionally, mechanically perturbed by micropipette aspiration or sonicated (40 kHz). The agarose solution once made was left in the water bath (65°C). The base medium was either, Opti-MEM® for HEK-293 derivative cell lines, DMEM-ITS for oMSC, osteoblasts and chondrocytes or NB-A+ for neurospheres.

ECM Protein Supplements

The ECM proteins were prepared by diluting from stock concentrations into an active gel form i.e. the working solution. Stock solutions of the ECM proteins were 5.0 mg/mL bovine collagen I, 1.0 mg/mL natural mouse laminin, and 1.0 mg/mL human fibronectin, and stored as aliquots at either, 4°C (collagen) or -20°C (laminin and fibronectin). The collagen I gel working solution (3.0 mg/mL) was prepared by mixing ice-cold reagents in the order: collagen I stock (50 μL), 10× concentrate PBS (8.3 μL), 1 N NaOH (1.3 μL), and ultrapure water (23.8 μL). Laminin and fibronectin working solutions (0.1 mg/mL) were made by diluting the respective stock solutions in PBS (typically 10 μL protein stock with 90 μL PBS). All ECM protein-working solutions were prepared just before their addition to the bioink, in a laminar flow biological safety cabinet.

**Table 1:** Agarose based bioink compositions.

| # | Scaffold Solution Type | Typical Scaffold Solution Composition | Cell ρ used (cells/mL) | Scaffold Phase Transferrable? |
|---|---|---|---|---|
| **SS1** | **Agarose with FMOC-XX (original)** | 89% V/V: 13-15 mg/mL agarose in Opti-MEM® or DMEM-ITS media<br>11% V/V: 10 mM FMOC-XX in ultrapure water | 5-15 × $10^6$ | Yes |
| **SS2** | **Agarose with FMOC-XX (diluted)** | 80% V/V: 13-15 mg/mL agarose in Opti-MEM® or DMEM-ITS<br>10% V/V: 10 mM FMOC-XX in ultrapure water<br>10% V/V: PBS | 1-15 × $10^6$ | Yes |
| **SS3** | **Agarose, FMOC-XX with ECM protein** | 80% V/V: 13-15 mg/mL agarose in Opti-MEM® or DMEM-ITS<br>10% V/V: 10 mM FMOC-XX in ultrapure water<br>10% V/V: ECM protein dissolved in PBS | 5-15 × $10^6$ | Yes |

***Resuspension of harvested cells as bioink:***

Cells were harvested and mixed into scaffold solutions as follows. For HEK-293T i.e. non adherent cells, a confluent T25 flask culture would be resuspended in Opti-MEM® (5 mL), an aliquot of this (typically 1-2 mL) was centrifuged (3-5 min, 300-500×G) in a 5702 centrifuge [*Eppendorf*] and the resulting pellet was resuspended in the scaffold solution at the desired cell density. For a typical resuspension, 100-200 μL of scaffold solution was mixed with cells to give a 15×$10^6$ cells/mL solution. Whereas, for adherent cell lines such as HEK-293/YFP, before an aliquot of cell solution is obtained, the culture was first trypsinised, and then re-suspended in Opti-MEM® (31985-062) post centrifugation. When MSC, osteoblast or chondrocyte bioinks were prepared the cells were re-suspended in DMEM-ITS media instead of Opti-MEM®. Neurosphere harvesting followed the same protocols as for their passaging, except instead of re-plating after the NB-A+ washes, the cells were centrifuged and re-suspended in the cell scaffold solution.

To re-suspend the cells at the desired density from a cell suspension aliquot, the cell density of the aliquot was first determined and the resuspension volume calculated using Equation 3.

This equation derives from the total cell number ($n_{cells}$) of a solution being equal to cell density ($\rho_{cells}$, in cells/mL) multiplied by volume ($V_{cells}$, in mL) Equation 1. For a resuspension, the total number of cells doesn't change, hence the cell number formula can used to equate the original and final volume solutions as Equation 2, which can be rearranged as Equation 3.

Equation 1 $$n_{cells} = \rho_{cells} \times V_{cells}$$

Equation 2 $$\rho_{initial} \times V_{initial} = \rho_{final} \times V_{final}$$

$$\text{Equation 3} \qquad \frac{\rho_{initial}}{\rho_{final}} \times V_{initial} = V_{final}$$

The standard way to determine the density of the cell aliquot was to mix 100 μL: 100 μL cell aliquot to trypan blue solution (dead cells only, stained dark blue) to produce a count solution. This solution (2× 10μL) was added to a disposable haemocytometer chip which is read by an automated cell counter [II FL, *Countess*] on either side of the chip to give two sets of values. The automated cell counter gives the live cell density, dead cell density and total cell density. The average of the two live cell density counts was used as the value for the initial cell density, and subsequently used to calculate the resuspension / final volume using Equation 3.

***3D bioprinting of the droplet networks:***

Droplet Printer Overview

A description of an example of an apparatus for generating droplets (e.g. a 3D-printer of droplet networks) can be found in Villar et al, Science 340, 48-52 (2013). In brief, the droplet generator (which may be referred to as "piezo") comprised a piezoelectric disc which seals the back of an aqueous chamber with a protruding tapered capillary nozzle. The piezo can eject droplets from the nozzle upon application of a square-wave voltage pulse when the tip is submerged in a bulk hydrophobic medium such as a lipid-in-oil solution (for instance as shown in Figure 1a or 1d). An electronic micromanipulator was used to move the printing stage, e.g. an oil container, in three dimensions. This in combination with lab-designed printing software that interprets "printing maps" and automates droplet ejection, allowed the construction of 3D droplet networks by successive layering of spatially assigned droplets. Laminar 3D structures produced by the process are shown in Figures 2e-h.

Printer Preparation

Before printing, the piezo's aqueous chamber, printing oil container, and other print items, were thoroughly cleaned with ultrapure water and ethanol, then dried under $N_{2\,(g)}$. On the day of use, capillaries were flushed with ultrapure water, ethanol and isopropanol and then dried under $N_{2\,(g)}$. Cleaned capillaries were subsequently vacuum-sealed in a plasma cleaner [Femto version A, *Diener Electronic*] and treated with oxygen plasma (8 min, 5-10 SCCM). With the instrument cleaned, the piezo was filled with ultrapure water and the capillary inserted.

In between each newly loaded bioink, the capillary was cleaned by soaking in Virkon then 8 M NaOH, and the aqueous chamber was replenished with lost water. For agarose-based bioinks the capillary was also cleaned by soaking in pure water at 65°C.

Printed Cell-Laden Droplet Networks

Cell-laden scaffolds were printed into 65:35 *V*:*V* mix of (undecane):(silicone oil) containing 1.2 mM DPhPC with an agarose based-scaffold solution containing cells (i.e. a bioink). The standard print protocol now follows.

The bioink was vortexed mixed and an aliquot (10 μL) was placed in the printer loading well array alongside hexadecane (8 μL). Hexadecane (~1 μL) was first suction loaded into the printer nozzle followed by the bioink (1-6 μL) (Figure 1d). For agarose-based bioinks, the outside tip of the glass capillary was wiped after loading with a lens tissue soaked in pure water.

Once the capillary was submerged into the print oil (usually 200 μL), the piezo was continually fired with varied voltage pulses until conditions were found for the reproducible ejection of singlet droplets, ideally of uniform size. Typical tuned pulse parameters were 50-350 μs pulse-width with voltages of 40-63 V.

The tuned pulse could be used to automate print cell-laden droplet networks, however. Typically, the voltage of this print pulse would be gradually increased throughout a print session of multiple networks to keep consistent droplet production. Generally, multiple networks were successively printed within the same print chamber, with the last network left to stand for 5 min before moving. Information on the bioink print order and print parameters are found below for the different network types.

Single Cell-Type Scaffolds

Cell scaffolds of a single cell-type were mainly printed as 2-4 layer droplet sheets with horizontal map dimensions ($x \times y$ pixels) of 7×9 or 7×8. For scaffolds (i.e. droplet assemblies) to be phased transferred, 4 layers was the usual thickness.

Junction Scaffolds (Two Cell-Types)

For all junction scaffolds, cell-laden bioink with or without collagen as printed using a single droplet generator to print cell-type 1 first, then cell-type 2 after nozzle cleaning and bioink loading. Each junction consisted of a wider basement droplet sheet (cell-type 1), on top of which, was a centrally aligned narrower droplet sheet (cell-type 2).

In all instances the junction networks were printed as follows, the lower droplet sheet was printed as a full map (i.e. a map where droplets were ejected at each pixel) for 3-4 layers and then sometimes the structures edges would be flattened by printing a hollow map (i.e. a map where droplets were ejected only at the edge of the map) for two further layers on top. The upper layer was printed similarly: a full map for 3-4 layers then sometimes a hollow map for two layers. The specific map sizes and number of layers printed for each junction are summarised in Table 2.

**Table 2:** Print details for the cell junction production. Each junction print is listed with bioink cell density, and the map dimensions (horizontal pixels $x \times y$ by vertical pixels $z$) for the upper and lower layers. Non-fluorescent cells were stained with red CMPTX (RC) or deep red (DR) cellTracker dye (Figure 2).

| **Printed Junction** | **Lower Cell** $\rho$ **(cells/ mL)** | **Upper Cell** $\rho$ **(cells/ mL)** | **Lower Print Dimensions** | | **Upper Print Dimensions** | |
|---|---|---|---|---|---|---|
| | | | **Full Map** $((x \times y) \times z)$ | **Hollow Map** $((x \times y) \times z)$ | **Full Map** $((x \times y) \times z)$ | **Hollow Map** $((x \times y) \times z)$ |
| *chondrocyte (DR) below osteoblasts (RC) [1]* | $3 \times 10^6$ | $1 \times 10^6$ | $(7 \times 9) \times 3$ | N/A | $(6 \times 8) \times 4$ | N/A |
| *chondrocyte (DR) below osteoblasts (RC) [2]* | $6 \times 10^6$ | $10 \times 10^6$ | $(7 \times 9) \times 4$ | $(7 \times 8) \times 2$ | $(6 \times 7) \times 4$ | N/A |
| *HEK-293/YFP below HEK-293/CFP (DR)* | $10 \times 10^6$ | $9 \times 10^6$ | $(7 \times 9) \times 3$ | $(7 \times 9) \times 2$ | $(6 \times 7) \times 4$ | N/A |
| *HEK-293T(RC) below HEK-293T (DR)* | $12 \times 10^6$ | $14 \times 10^6$ | $(7 \times 8) \times 3$ | $(7 \times 9) \times 2$ | $(6 \times 7) \times 3$ | $(6 \times 7) \times 2$ |

Pathway Networks (Two Cell-Types)

For all pathway scaffolds, cell-laden bioink with or without collagen were created using a single droplet generator to first print cell-type 1, then cell-type 2 after nozzle cleaning and bioink loading. Each pathway network consisted of a narrow pathway architecture (cell-type1), surrounded in the same plane and above by structural droplets (cell-type 2).

In all instances pathway networks were printed as follows, the pathway droplets were printed first for 3-7 layers, after which, the pathway plane, structural droplets were printed for 2-6 layers and finally the network would generally be finished with 2 layers of topping structural droplets. The map dimension for each of these 3 maps would stay constant throughout the network print, but the design varied between pathway networks (see Table 3).

**Table 3:** Print details for production of cell pathway structures. Each pathway print is listed with bioink cell density, map dimensions (horizontal pixels $x \times y$) and no. of layers printed (vertical pixel z) and the width of the pathway (pixel width in map). The cells used for each structure were: HEK-293/YFP (path) and HEK-293/CFP (structure) in trifurcated pathway [1]; HEK-293/CFP (path) and HEK-293/YFP (structure) in trifurcated pathway [2]; and HEK-293T (RC) (path) and HEK-293T (DR) (structure) in trifurcated pathway [1]. Non-fluorescent cells were stained with red CMPTX (RC) or deep red (DR) cellTracker dye.

| **Printed Junction** | **Pathway Cell** $\rho$ **(cells/mL)** | **Structural Cell** $\rho$ **(cells/mL)** | **Map Dimensions** $(x \times y)$ | **Printed Layers** | | | **Pathway Width (pixel)** |
|---|---|---|---|---|---|---|---|
| | | | | **Pathway** (z) | **Sides** (z) | **Top** (z) | |
| *trifurcated pathway [1]* | $14 \times 10^6$ | $14 \times 10^6$ | $13 \times 13$ | 5 | 5 | 0 | 3 |
| *trifurcated pathway [2]* | $14 \times 10^6$ | $14 \times 10^6$ | $10 \times 12$ | 5 or 7 | 6 or 5 | 2 | 2 |
| *bifurcated Pathway* | $10 \times 10^6$ | $13 \times 10^6$ | $8 \times 9$ | 3 or 4 | 2 | 2 | 1 |

Mixed-Cell Printed Network

The scaffolds containing mixed cells throughout was printed with a bioink comprising a 1:1 *V*:*V* mix of 10×10$^6$ HEK-293T (red CMPTX stained)/mL in bioink plus collagen, with 13×10$^6$ HEK-293T (deep red stained)/mL in bioink plus collagen. These were printed as a horizontal 7×8 (*x*×*y*) map for 4 layers.

***Network gelation and gel coating (phase transfer preparation):***

Scaffold Gelation

Printed scaffolds comprised of either **SS7** or **SS8** bound in *standard bioink print oil* were left to rest (5 min) post print. The scaffolds were then gelled in a fridge (4 °C, 20-25 min). Gelled networks were either used straight away or stored in a hydration chamber. After gelling, the networks (i.e. droplet assemblies) were transferred to an aqueous phase such as culture medium.

***Cell scaffold Maintenance***

The phase-transferred scaffolds were cultured in culture medium (~600 μL per well) for up to 14 days using a microscope chamber slides [154534K, Lab-Tek™] container. Scaffolds were stored between media exchanges and experimentation in a Midi 40 cell incubator [*Thermo Scientific*] set at 37 °C with 5% $CO_{2(g)}$. Every 2-3 days the scaffolds media was exchanged, for a single well this proceeded as follows: ~200-300 μL container medium was carefully removed near the air-medium interface and then 300 μL fresh medium was added slowly at corner of the well, this removal and replacement of medium was repeated 1-2 more time(s). Media exchanges were performed in a laminar flow biological safety cabinet.

The majority of HEK-293T and fluorescent HEK-293 scaffolds were cultured in fully supplemented *standard cell scaffold culture* medium.

The oMSC scaffolds were cultured in *MSC culture* medium for standard viability assessment, and for differentiation experiments *MSC differentiation* medium or *MSC control* medium was used.

The neurosphere scaffolds were cultured in either *standard cell scaffold culture* medium or *neurosphere culture* medium.

***Additional Cell Culture Media:***

Cell-Culture Media Mixtures

Each cell type has its own specific cell culture medium composition. All culture media were composed of essential medium with or without the following supplements: 10% *V*/*V* FBS, 2 mM GlutaMAX™ (which is a source of glutamine), 0.1 mM MEM non-essential amino acids, 10-25 mM HEPES (buffering agent), 100 U/mL penicillin (an antibiotic) and 100 μg/mL streptomycin (an antibiotic). The ratio of these components can be seen in Table 4.

The base medium varied between the different cell lines and was either DMEM, MEM or Neurobasal®-A. Specifically three different DMEM culture media were used throughout the culture of most cell lines, these are now described along with their *Sigma-Aldrich* product codes: DMEM D6546 was, a high glucose medium, and used for all HEK-293 cell-line derivatives; DMEM D5564 was, a low glucose medium, and used in the culture of MSCs; whilst DMEM D5671 was, a high glucose medium with no sodium pyruvate, and used to make DMEM-ITS base medium for the differentiation experiments with the MSCs. Each DMEM contained either high (4500 mg/L) or low (1000 mg/mL) glucose with sodium bicarbonate, pyridoxine hydrochloride and 10 mg/L sodium pyruvate (unless stated). For osteogenic differentiation of MSCs and the culture of osteoblasts, MEM M4526 was used, and contained sodium bicarbonate and increased amino acid concentrations compared to standard MEM. Neuronal cells were cultured in Neurobasal®-A (10888-022), a serum-free neuronal medium . The base medium of each cell-line's culture medium is also described in Table 4.

Additional nutrients or special antibiotics were also used for all cell-lines except for HEK-293T. These are described below for each cell type.

**Table 4:** Compositions of standard culture media used to grow the various cell-lines. The 100× concentrated stocks are 200 mM L-alanyl-L-glutamine (GlutaMAX™), 10 mM MEM-NEAA, and 10,000 U/mL penicillin with 10,000 μg/mL streptomycin (PenStrep).

| **Medium Type** | **Media Compositions** | | | | | | |
|---|---|---|---|---|---|---|---|
| | **Base Medium** | **FBS (% *V/V*)** | **GlutaMAX™ (100×) (% *V/V*)** | **100× MEM-NEAA (% *V/V*)** | **1 M HEPES (% *V/V*)** | **100× PenStrep (% *V/V*)** | **Additional Nutrients** |
| *HEK-293T Culture* | DMEM D6546 | 10 | 1 | 0 | 0 | 0 | No |
| *HEK-293/xFP Culture* | DMEM D6546 | 10 | 1 | 1 | 0 | 0 | No |
| *Original Cell Scaffold Culture* | DMEM D6546 | 10 | 1 | 0 | 0 | 0 | No |
| *Standard Cell Scaffold Culture* | DMEM D6546 | 10 | 1 | 1 | 1 | 1 | No |

Fluorescent HEK-293 Culture Medium

For the culture of fluorescent protein expressing HEK-293 cells, 10 μg/mL blasticidin was also added. Stock blasticidin solution (10 mg/mL) was prepared by dissolving blasticidin powder (50 mg) in filter-sterilised ultrapure water (5 mL) and stored as 10 μL aliquots at - 20 °C. The stock solution was added at 1 μL/mL per culture flask.

Ovine MSC Differentiation Media

For MSCs differentiation experiments, the cells were cultured in MSC differentiation media or MSC control media, the base media of which is ITS-DMEM comprised of DMEM D5671 un-supplemented with FBS, but containing ITS (at 10.0 mg/mL bovine insulin, 5.5 mg/mL human transferrin and 6.7 μg/mL sodium selenite), 1 mM sodium pyruvate, 2 mM GlutaMAX™, 100 U/mL penicillin and 100 μg/mL streptomycin. The MSC differentiation media for day 0-7 also contained 100 nM dexamethasone, 80 μM ascorbic acid-2-phosphate and 10 ng/mL TGF-β3, all freshly supplemented. After day 7, the MSC differentiation media additionally included 10 ng/mL insulin, also added fresh. The MSC control media is the same as MSC differentiation media for day 0-7, but without the TGF-β3. A summary of additional nutrient supplements for MSC culture can be seen in Table 5.

The additional nutrients, dexamethasone, ascorbic acid, TGFβ3 and insulin, were all prepared from powdered reagents as stock solutions which were vortex mixed and then filter-sterilised and stored as aliquots at -20 °C. The 100 μM dexamethasone stock was prepared by dissolving dexamethasone (3.925 mg) in ethanol (1 mL) and then further diluted to a 0.01% (*V/V*) solution in ITS-DMEM (1 mL). For ascorbate, the 80 mM stock was made by dissolving L-ascorbic acid 2-phosphate sesquimagnesium salt hydrate in ultrapure water (3 mL). The 10 μg/mL TGF-β3 stock was created by dissolving TGF-β3 in filter sterilised 4 mM hydrochloric acid containing 1 mg/mL BSA. Finally, the 10 mg/mL stock insulin was prepared by dissolving insulin (20 mg) in ultrapure water diluted acetic acid (2 mL, pH 2.0).

**Table 5:** Proportion of additional nutrient supplements added to the MSC media. The nutrients added are from stock solutions which are as follows, 100× concentrated ITS (at 1.00 g/mL bovine insulin, 0.55 g/mL human transferrin and 0.67 mg/mL sodium selenite), 100× concentrate sodium pyruvate (100 mM), 10 μg/mL FGF, 100 μM dexamethasone, 80 mM ascorbic acid-2-phosphate, 10 μg/mL TGF-β3 and 10 mg/mL insulin.

| | **Proportion of Additional Nutrient Supplements** | | | | | | |
|---|---|---|---|---|---|---|---|
| **Media Type** | **ITS** (% *V/V*) | **Sodium Pyruvate** (% *V/V*) | **FGF** μL/mL | **Ascorbic Acid** μL/mL | **Dexamethasone** μL/mL | **TGF -β3** μL/ mL | **Insulin** μL/mL |
| *Chondrocyte IMSC Culture* | 0 | 1 | 0.5 | 0.0 | 0.0 | 0.0 | 0.0 |
| *MSC Differentiation (day 0-7)* | 1 | 1 | 0.0 | 1.0 | 1.0 | 1.0 | 0.0 |
| *MSC Differentiation (day> 7)* | 1 | 1 | 0.0 | 1.0 | 1.0 | 1.0 | 1.0 |
| *MSC Control* | 1 | 1 | 0.0 | 1.0 | 1.0 | 0.0 | 0.0 |

Other possible culture media include the following.

Osteoblast Culture Medium

For MSC osteogenesis, the osteoblast culture medium also contained 50 μL/mL StemXVivo™ osteogenic supplement, which was added as supplied on the day of use. Osteogenic supplement was stored as aliquots at -20 °C.

Neurosphere Culture Medium

The neurospheres were cultured in neurosphere culture medium called NB-A+, which comprised Neurobasal®-A medium supplemented with 4% *V/V* B-27® supplement, 100 U/mL penicillin and 100 μg/mL streptomycin. For neuronal stem cell expansion, NB-A+ was additionally supplemented with 20 ng/mL epidermal growth factor and 20 ng/mL basic FGF.

Ovine MSC and Chondrocyte Culture Medium

All oMSC media involved additional supplements; for the MSC culture, media also contained 5 ng/mL FGF, added just before time of use from a 10 μg/mL FGF stock. Chondrocyte culture medium was exactly the same as the MSC culture medium. Stock 10 μg/mL FGF solution was made by dissolving FGF (50 μg) in filter-sterilised 5 mM Tris HCl solution (5 mL, pH 7.6), with aliquots stored at -20°C.

***Advantages of process of the invention compared to other printing technologies***

The process of the invention as described in the Example above has several advantages when compared to known printing technologies.

Higher droplet resolution compared to valve-based droplet bioprinting

Valve-based bioprinters can make liquid hydrogel droplets on demand and stack these to form 3D scaffolds. Typically, however, their droplet resolution (printer voxel resolution) is much lower i.e. an order of magnitude lower than our technique. Li et al., Angew. Chemie Int. Ed. 54, 1-6a (2015) produces droplets of diameter of 500 μm and volume 65 nL. Gurkan et al., Mol. Pharm. 11, 2151-9 (2014) produces droplets of diameter 300 μm and volume 14 nL. The smallest micropipetted droplet typically has a volume of 200 nL and diameter ~0.7 mm. The process of the invention allows the production of droplets of a salt-based solution with a diameter of 50 μm and volume of 65 pL and droplets of bioink having a diameter of -130 μm and a volume of~1.2 nL.

High cell density for a printed hydrogel scaffold

Most known bioprinting techniques are performed with cell densities at the $10^6$ cells/mL scale. The process of the invention allows printing at a density an order of magnitude greater (e.g. $15\times10^6$ cells/mL).

High viability in scaffold

The cell viability post printing is between 80-95% as shown in Figure 3c

Uniform cell distribution throughout the structure

The cells are uniformly distributed with the process of the invention as shown in Figure 1e.

No supporting scaffold necessary

Some bioprinting methods use a secondary supporting scaffold to offer rigidity to, or ensure porosity within, their cell-laden structure (Derby, Science 338, 921-6, 2012). For a tissue to grow it is likely that this supporting scaffold would have to degrade which is a difficult material design constraint. Also, such a scaffold, if implanted would have to be biocompatible with the host and avoid rejection by the immune system. The hydrogel-based cell-laden scaffolds do not require such a supporting scaffold.

High pattern resolution

Complex architectures of multiple cell types such as cross pathways, arborised pathways (vasculature or nervous tissue like structures) and network junctions have been printed with high feature resolution (~250 μm i.e. 2 droplet width) by the process of the invention. This is a greater pattern resolution than with known methods (e.g. Kolesky et al., Adv. Mater. 26, 3124-30, 2014). This high pattern resolution is shown in Figure 2.

Reproducible fabrication of mm scale microtissues

No papers have shown the reproducible production of small microtissues of the dimensions demonstrated by the process of the invention (typically $1\times1\times0.4$ $mm^3$). Most known techniques show scaffolds on the cm scale.

Scaffold fabrication is unaffected by ECM supplementation

Using the process of the invention it is possible to supplement the bioink with proportions of extracellular matrix proteins such as collagen, fibronectin and laminin without affecting the printing process. It has been empirically noticed that a low proportion of ECM is enough to aid initial cell proliferation. As such, it should be feasible to supplement the bioink with alternative ECM-based supplements.

## Claims

1. A process for producing a droplet assembly, which droplet assembly comprises a plurality of droplets, wherein each of said droplets comprises:

    an aqueous medium comprising (i) a hydrogel compound, which hydrogel compound is a polysaccharide, and (ii) one or more stabilising agents, which one or more stabilizing agents are selected from compounds which comprise a dipeptide; and
    one or more biological cells disposed in the aqueous medium,

    which process comprises:
    generating, in a bulk hydrophobic medium comprising one or more amphipathic compounds, a plurality of droplets, wherein each of said droplets comprises:

    an aqueous medium comprising (i) a hydrogel compound, which hydrogel compound is a polysaccharide, and (ii) one or more stabilising agents, which one or more stabilizing agents are selected from compounds which comprise a dipeptide; and
    one or more biological cells disposed in the aqueous medium.

**2.** A process according to claim 1, wherein:

the hydrogel compound is agarose; and/or
the one or more stabilising agents are selected from dipeptides protected with a FMOC group, preferably wherein the one or more stabilising agents are FMOC-isoleucine-glycine and/or FMOC-phenylalanine-phenylalanine.

**3.** A process according to claim 1 or claim 2, wherein:

(A) the aqueous medium further comprises an extracellular membrane protein, preferably wherein the extracellular membrane protein comprises collagen, fibronectin or laminin;
(B) the aqueous medium further comprises a culture medium, preferably wherein the culture medium is an aqueous solution of one or more amino acids, one or more salts, glucose, and one or more vitamins; and/or
(C) the aqueous medium comprises:

(a) a hydrogel at a concentration of from 0.5 to 30.0 mg/mL;
(b) a culture medium at a concentration of from 60.0 to 90.0 volume%; and
(c) a compound comprising a dipeptide at a concentration of from 0.1 to 10.0 mmol/L; and optionally
(d) an extracellular membrane protein at a concentration of from 0.001 to 1.0 mg/mL.

**4.** A process according to any one of the preceding claims, wherein the one or more biological cells:

(A) are selected from two or more different types of biological cells, optionally wherein

(a) one or more of the droplets each comprises two or more biological cells disposed in the aqueous medium, and where the two or more biological cells are selected from two or more different types of biological cells, or
(b) the droplet assembly comprises

a first plurality of droplets, each of which droplets comprises one or more of a first type of biological cells disposed in the aqueous medium, and
a second plurality of droplets, each of which droplets comprises one or more of a second type of biological cells disposed in the aqueous medium;

(B) are mammalian cells; and/or
(C) are disposed in the aqueous medium at a concentration of from $10^5$ to $10^9$ cells per mL of aqueous medium.

**5.** A process according to any one of the preceding claims, wherein the bulk hydrophobic medium comprises a hydrocarbon compound and/or a silicone oil, preferably wherein the hydrocarbon compound is a $C_8$ to $C_{16}$ alkane, more preferably wherein the hydrocarbon compound is undecane;

optionally wherein the bulk hydrophobic medium comprises a mixture of a hydrocarbon and a silicone oil in a ratio (hydrocarbon):(silicone oil) of from 50:50 to 80:20 by volume, preferably in a ratio of from 60:40 to 70:30 by volume; and
optionally wherein the one or more amphipathic compounds are selected from lipids, preferably wherein the one or more amphipathic compounds are selected from phospholipids, more preferably wherein the one or more amphipathic compounds are selected from phosphocholine lipids.

**6.** A process according to any one of the preceding claims, wherein generating said plurality of droplets comprises:

(i) a plurality of dispensing steps, each of which dispensing steps comprises injecting into the bulk hydrophobic medium a volume of the aqueous medium with the one or more biological cells disposed therein, optionally wherein the volume of the aqueous medium with the one or more biological cells disposed therein is from 0.001 to 100 nL, and optionally wherein generating said plurality of droplets comprises 100 or more of the dispensing steps;
and/or
(ii) using an apparatus for generating droplets,

which apparatus comprises

(a) at least one droplet generator;
(b) a container which is movable relative to the at least one droplet generator; and
(c) a control unit which is adapted to control the dispensing of droplets from the at least one droplet generator and to control the movement of the container relative to the at least one droplet generator,

optionally wherein:

the or each droplet generator comprises:

a chamber for holding the aqueous medium with the one or more biological cells disposed therein;
an outlet; and
a component for displacing through said outlet a volume of the aqueous medium with the one or more biological cells disposed therein and thereby dispensing said volume as a droplet, optionally wherein the component is a piezoelectric transducer; and/or

the container which is movable relative to the at least one droplet generator comprises the bulk hydrophobic medium.

7. A process according to any one of the preceding claims, wherein at least one of the droplets further comprises an outer layer of amphipathic molecules around the surface of the aqueous medium, and preferably contacts another one of said droplets which further comprises an outer layer of amphipathic molecules around the surface of the aqueous medium wherein a layer of amphipathic molecules is formed between the first and second droplets as an interface, optionally wherein:

(A) the droplet assembly comprises a plurality of droplets arranged in a three-dimensional structure wherein each droplet in the three dimensional structure comprises an outer layer of amphipathic molecules around the surface of the aqueous medium and wherein each droplet in the three dimensional structure contacts at least one other droplet in the three dimensional structure forming a layer of amphipathic molecules as an interface between contacting droplets;
(B) the layer of amphipathic molecules formed between the first and second droplets as an interface is a bilayer of amphipathic molecules formed between the first and second droplets as an interface; and/or
(C) the outer layer of amphipathic molecules around the surface of the aqueous medium in one or more of the droplets further comprises one or more transmembrane proteins.

8. A process according to any one of the preceding claims, which process further comprises:

(A) before the step of generating a plurality of droplets, a step of preparing the aqueous medium with one or more biological cells disposed therein, which preparation step comprises providing an aqueous medium and suspending a plurality of biological cells in the aqueous medium;
(B) a gelling step, which gelling step comprises allowing the aqueous medium comprising the hydrogel compound to form a gel, preferably wherein the gelling step comprises cooling the droplet assembly to a temperature of less than or equal to 10.0°C and allowing the aqueous medium comprising the hydrogel compound to form a gel;
(C) culturing the plurality of cells to produce a celluralised droplet assembly; and/or
(D) isolating the droplet assembly from the bulk hydrophobic medium.

9. A droplet assembly comprising a plurality of droplets, wherein each of said droplets comprises:

(i) an aqueous medium comprising (i) a hydrogel compound, which hydrogel compound is a polysaccharide, and (ii) one or more stabilising agents, which one or more stabilizing agents are selected from compounds which comprise a dipeptide;
(ii) one or more biological cells disposed in the aqueous medium; and
(iii) an outer layer of amphipathic molecules around the surface of the aqueous medium,

wherein at least one droplet in the droplet assembly contacts at least one other droplet in the droplet assembly forming a layer of amphipathic molecules as an interface between contacting droplets.

10. A droplet assembly according to claim 9, wherein the hydrogel compound is agarose.

**11.** A droplet assembly according to claim 9 or claim 10 wherein the one or more stabilising agents are selected from dipeptides protected with a FMOC group, preferably wherein the one or more stabilising agents are FMOC-isoleucine-glycine and/or FMOC-phenylalanine-phenylalanine.

**12.** A droplet assembly according to any one of claims 9 to 11 wherein the aqueous medium comprises an extracellular membrane protein, preferably wherein the extracellular membrane protein comprises collagen, fibronectin or laminin.

**13.** A droplet assembly according to any one of claims 9 to 12, wherein:

(A) the droplet assembly comprises:

a first plurality of droplets, each of which droplets comprises one or more of a first type of biological cells disposed in the aqueous medium, and
a second plurality of droplets, each of which droplets comprises one or more of a second type of biological cells disposed in the aqueous medium;

(B) each of the plurality of droplets has a volume of from 0.001 to 100 nL;
(C) the droplet assembly is as further defined in claim 3; and/or
(D) the aqueous medium comprising a hydrogel compound is in the form of a gel.

**14.** A composition comprising a bulk hydrophobic medium and, disposed in the bulk hydrophobic medium, a droplet assembly as defined in any one of claims 9 to 13.

**15.** A droplet assembly obtainable by a process as defined in any one of claims 1 to 8.

**Patentansprüche**

**1.** Verfahren zum Herstellen einer Tröpfchenanordnung, wobei die Tröpfchenanordnung eine Mehrzahl von Tröpfchen umfasst, wobei jedes der Tröpfchen umfasst:

ein wässriges Medium, umfassend (i) eine Hydrogelverbindung, wobei die Hydrogelverbindung ein Polysaccharid ist, und (ii) ein oder mehrere Stabilisierungsmittel, wobei das eine oder die mehreren Stabilisierungsmittel aus Verbindungen ausgewählt sind, die ein Dipeptid umfassen; und
eine oder mehrere biologische Zellen, die in dem wässrigen Medium angeordnet sind, wobei das Verfahren umfasst:
Erzeugen einer Mehrzahl von Tröpfchen in einem hydrophoben Massenmedium, das eine oder mehrere amphipathische Verbindungen umfasst, wobei jedes der Tröpfchen umfasst:

ein wässriges Medium, umfassend (i) eine Hydrogelverbindung, wobei die Hydrogelverbindung ein Polysaccharid ist, und (ii) ein oder mehrere Stabilisierungsmittel, wobei das eine oder die mehreren Stabilisierungsmittel aus Verbindungen ausgewählt sind, die ein Dipeptid umfassen; und
eine oder mehrere biologische Zellen, die in dem wässrigen Medium angeordnet sind.

**2.** Verfahren nach Anspruch 1, wobei:

die Hydrogelverbindung Agarose ist; und/oder
das eine oder die mehreren Stabilisierungsmittel aus Dipeptiden ausgewählt sind, die mit einer FMOC-Gruppe geschützt sind, wobei das eine oder die mehreren Stabilisierungsmittel vorzugsweise FMOC-Isoleucin-Glycin und/oder FMOC-Phenylalanin-Phenylalanin sind.

**3.** Verfahren nach Anspruch 1 oder 2, wobei:

(A) das wässrige Medium ferner ein extrazelluläres Membranprotein umfasst, wobei das extrazelluläre Membranprotein vorzugsweise Kollagen, Fibronektin oder Laminin umfasst;
(B) das wässrige Medium ferner ein Kulturmedium umfasst, wobei das Kulturmedium vorzugsweise eine wässrige Lösung von einer oder mehreren Aminosäuren, einem oder mehreren Salzen, Glucose und einem oder mehreren Vitaminen ist; und/oder

(C) das wässrige Medium umfasst:

(a) ein Hydrogel in einer Konzentration von 0,5 bis 30,0 mg/ml;
(b) ein Kulturmedium in einer Konzentration von 60,0 bis 90,0 Vol.-%; und
(c) eine Verbindung, die ein Dipeptid in einer Konzentration von 0,1 bis 10,0 mmol/l umfasst; und optional
(d) ein extrazelluläres Membranprotein in einer Konzentration von 0,001 bis 1,0 mg/ml.

**4.** Verfahren nach einem der vorhergehenden Ansprüche, wobei die eine oder die mehreren biologischen Zellen:

(A) aus zwei oder mehr verschiedenen Arten von biologischen Zellen ausgewählt sind, wobei optional

(a) ein oder mehrere der Tröpfchen jeweils zwei oder mehr biologische Zellen umfassen, die in dem wässrigen Medium angeordnet sind, und wobei die zwei oder mehr biologischen Zellen aus zwei oder mehr verschiedenen Arten von biologischen Zellen ausgewählt sind, oder
(b) die Tröpfchenanordnung umfasst

eine erste Mehrzahl von Tröpfchen, wobei jedes dieser Tröpfchen eine oder mehrere biologische Zellen einer ersten Art umfasst, die in dem wässrigen Medium angeordnet sind, und
eine zweite Mehrzahl von Tröpfchen, wobei jedes dieser Tröpfchen eine oder mehrere biologische Zellen einer zweiten Art umfasst, die in dem wässrigen Medium angeordnet sind;

(B) Säugetierzellen sind; und/oder
(C) in dem wässrigen Medium in einer Konzentration von $10^5$ bis $10^9$ Zellen pro ml des wässrigen Mediums angeordnet sind.

**5.** Verfahren nach einem der vorhergehenden Ansprüche, wobei das hydrophobe Massenmedium eine Kohlenwasserstoffverbindung und/oder ein Silikonöl umfasst, wobei die Kohlenwasserstoffverbindung vorzugsweise ein $C_8$ bis $C_{16}$-Alkan ist, stärker bevorzugt wobei die Kohlenwasserstoffverbindung Undecan ist;

wobei das hydrophobe Massenmedium optional ein Gemisch aus einem Kohlenwasserstoff und einem Silikonöl in einem Verhältnis (Kohlenwasserstoff) : (Silikonöl) von 50 : 50 bis 80 : 20 nach Volumen, vorzugsweise in einem Verhältnis von 60 : 40 bis 70 : 30 nach Volumen umfasst; und
wobei optional die eine oder die mehreren amphipathischen Verbindungen aus Lipiden ausgewählt sind, wobei die eine oder die mehreren amphipathischen Verbindungen vorzugsweise aus Phospholipiden ausgewählt sind, stärker bevorzugt wobei die eine oder die mehreren amphipathischen Verbindungen vorzugsweise aus Phosphocholinlipiden ausgewählt sind.

**6.** Verfahren nach einem der vorhergehenden Ansprüche, wobei das Erzeugen der Mehrzahl von Tröpfchen umfasst:

(i) eine Mehrzahl von Abgabeschritten, wobei jeder der Abgabeschritte das Einspritzen eines Volumens des wässrigen Mediums mit der einen oder den mehreren darin angeordneten biologischen Zellen in das hydrophobe Massenmedium umfasst, wobei optional das Volumen des wässrigen Mediums mit der einen oder den mehreren darin angeordneten biologischen Zellen 0,001 bis 100 nL beträgt, und wobei optional das Erzeugen der Mehrzahl von Tröpfchen 100 oder mehr der Abgabeschritte umfasst;
und/oder
(ii) Verwenden einer Einrichtung zum Erzeugen von Tröpfchen,

wobei die Einrichtung umfasst

(a) wenigstens einen Tröpfchengenerator;
(b) einen Behälter, der relativ zu dem wenigstens einen Tröpfchengenerator beweglich ist; und
(c) eine Steuereinheit, die angepasst ist, das Abgeben von Tröpfchen aus dem wenigstens einen Tröpfchengenerator zu steuern und die Bewegung des Behälters relativ zu dem wenigstens einen Tröpfchengenerator zu steuern,

wobei optional:

der oder jeder Tröpfchengenerator umfasst:

eine Kammer zum Halten des wässrigen Mediums mit der einen oder den mehreren darin angeordneten biologischen Zellen;
einen Auslass; und
eine Komponente zum Verdrängen eines Volumens des wässrigen Mediums mit der einen oder den mehreren darin angeordneten biologischen Zellen durch den Auslass und dadurch Abgeben des Volumens als ein Tröpfchen, wobei die Komponente optional ein piezoelektrischer Wandler ist; und/oder

der Behälter, der relativ zu dem wenigstens einen Tröpfchengenerator beweglich ist, das hydrophobe Volumenmedium umfasst.

**7.** Verfahren nach einem der vorhergehenden Ansprüche, wobei wenigstens eines der Tröpfchen ferner eine äußere Schicht aus amphipathischen Molekülen um die Oberfläche des wässrigen Mediums herum umfasst und vorzugsweise ein anderes der Tröpfchen berührt, das ferner eine äußere Schicht aus amphipathischen Molekülen um die Oberfläche des wässrigen Mediums herum umfasst, wobei eine Schicht aus amphipathischen Molekülen zwischen dem ersten und dem zweiten Tröpfchen als eine Grenzfläche ausgebildet wird, wobei optional:

(A) die Tröpfchenanordnung eine Mehrzahl von Tröpfchen umfasst, die in einer dreidimensionalen Struktur angeordnet sind, wobei jedes Tröpfchen in der dreidimensionalen Struktur eine äußere Schicht aus amphipathischen Molekülen um die Oberfläche des wässrigen Mediums herum umfasst und wobei jedes Tröpfchen in der dreidimensionalen Struktur wenigstens ein anderes Tröpfchen in der dreidimensionalen Struktur berührt und eine Schicht aus amphipathischen Molekülen als eine Grenzfläche zwischen sich berührenden Tröpfchen ausbildet;
(B) die Schicht aus amphipathischen Molekülen, die zwischen dem ersten und dem zweiten Tröpfchen als eine Grenzfläche ausgebildet ist, eine Doppelschicht aus amphipathischen Molekülen ist, die zwischen dem ersten und dem zweiten Tröpfchen als eine Grenzfläche ausgebildet ist; und/oder
(C) die äußere Schicht aus amphipathischen Molekülen um die Oberfläche des wässrigen Mediums in einem oder mehreren der Tröpfchen ferner ein oder mehrere Transmembranproteine umfasst.

**8.** Verfahren nach einem der vorhergehenden Ansprüche, wobei das Verfahren ferner umfasst:

(A) vor dem Schritt des Erzeugens einer Mehrzahl von Tröpfchen einen Schritt des Vorbereitens des wässrigen Mediums mit einer oder mehreren darin angeordneten biologischen Zellen, wobei der Vorbereitungsschritt das Bereitstellen eines wässrigen Mediums und das Suspendieren einer Mehrzahl von biologischen Zellen in dem wässrigen Medium umfasst;
(B) einen Gelierschritt, wobei der Gelierschritt Ermöglichen umfasst, dass das wässrige Medium, das die Hydrogelverbindung umfasst, ein Gel ausbildet, wobei der Gelierschritt vorzugsweise das Abkühlen der Tröpfchenanordnung auf eine Temperatur von weniger als oder gleich 10,0 °C und das Ermöglichen umfasst, dass das wässrige Medium, das die Hydrogelverbindung umfasst, ein Gel ausbildet;
(C) Kultivieren der Mehrzahl von Zellen zum Produzieren einer zellularisierten Tröpfchenanordnung; und/oder
(D) Isolieren der Tröpfchenanordnung von dem hydrophoben Massenmedium.

**9.** Tröpfchenanordnung, umfassend eine Mehrzahl von Tröpfchen, wobei jedes der Tröpfchen umfasst:

(i) ein wässriges Medium, umfassend (i) eine Hydrogelverbindung, wobei die Hydrogelverbindung ein Polysaccharid ist, und (ii) ein oder mehrere Stabilisierungsmittel, wobei das eine oder die mehreren Stabilisierungsmittel aus Verbindungen ausgewählt sind, die ein Dipeptid umfassen;
(ii) eine oder mehrere biologische Zellen, die in dem wässrigen Medium angeordnet sind; und
(iii) eine äußere Schicht aus amphipathischen Molekülen um die Oberfläche des wässrigen Mediums,

wobei wenigstens ein Tröpfchen in der Tröpfchenanordnung wenigstens ein anderes Tröpfchen in der Tröpfchenanordnung berührt und eine Schicht amphipathischer Moleküle als eine Grenzfläche zwischen sich berührenden Tröpfchen ausbildet.

**10.** Tröpfchenanordnung nach Anspruch 9, wobei die Hydrogelverbindung Agarose ist.

**11.** Tröpfchenanordnung nach Anspruch 9 oder 10, wobei das eine oder die mehreren Stabilisierungsmittel aus Dipeptiden ausgewählt sind, die mit einer FMOC-Gruppe geschützt sind, wobei das eine oder die mehreren Stabilisierungsmittel vorzugsweise FMOC-Isoleucin-Glycin und/oder FMOC-Phenylalanin-Phenylalanin sind.

**12.** Tröpfchenanordnung nach einem der Ansprüche 9 bis 11, wobei das wässrige Medium ein extrazelluläres Membranprotein umfasst, wobei das extrazelluläre Membranprotein vorzugsweise Kollagen, Fibronektin oder Laminin umfasst.

**13.** Tröpfchenanordnung nach einem der Ansprüche 9 bis 12, wobei:

(A) die Tröpfchenanordnung umfasst:

eine erste Mehrzahl von Tröpfchen, wobei jedes der Tröpfchen eine oder mehrere einer ersten Art von biologischen Zellen umfasst, die in dem wässrigen Medium angeordnet sind, und
eine zweite Mehrzahl von Tröpfchen, wobei jedes dieser Tröpfchen eine oder mehrere einer zweiten Art von biologischen Zellen umfasst, die in dem wässrigen Medium angeordnet sind;

(B) jedes der Mehrzahl von Tröpfchen ein Volumen von 0,001 bis 100 nL aufweist;
(C) die Tröpfchenanordnung ferner wie in Anspruch 3 definiert ist; und/oder
(D) das wässrige Medium, das eine Hydrogelverbindung umfasst, in der Form eines Gels ist.

**14.** Zusammensetzung, umfassend ein hydrophobes Massenmedium und eine in dem hydrophoben Massenmedium angeordnete Tröpfchenanordnung nach einem der Ansprüche 9 bis 13.

**15.** Tröpfchenanordnung, die durch ein Verfahren nach einem der Ansprüche 1 bis 8 erhalten werden kann.

## Revendications

**1.** Procédé pour produire un ensemble de gouttelettes, lequel ensemble de gouttelettes comprend une pluralité de gouttelettes, dans lequel chacune desdites gouttelettes comprend :

un milieu aqueux comprenant (i) un composé hydrogel, lequel composé hydrogel est un polysaccharide, et (ii) un ou plusieurs agents stabilisants, lequel un ou lesquels plusieurs agents stabilisants sont sélectionnés parmi des composés qui comprennent un dipeptide ; et
une ou plusieurs cellules biologiques disposées dans le milieu aqueux,
ledit procédé comprend :
la génération, dans un milieu hydrophobe en masse comprenant un ou plusieurs composés amphipathiques, d'une pluralité de gouttelettes, dans lequel chacune desdites gouttelettes comprend :

un milieu aqueux comprenant (i) un composé hydrogel, lequel composé hydrogel est un polysaccharide, et (ii) un ou plusieurs agents stabilisants, lequel un ou lesquels plusieurs agents stabilisants sont sélectionnés parmi des composés qui comprennent un dipeptide ; et
une ou plusieurs cellules biologiques disposées dans le milieu aqueux.

**2.** Procédé selon la revendication 1, dans lequel :

le composé hydrogel est de l'agarose ; et/ou
l'un ou les plusieurs agents stabilisants sont sélectionnés parmi des dipeptides protégés avec un groupe FMOC, de préférence dans lequel l'un ou les plusieurs agents stabilisants sont FMOC-isoleucine-glycine et/ou FMOC-phénylalanine-phénylalanine.

**3.** Procédé selon la revendication 1 ou la revendication 2, dans lequel :

(A) le milieu aqueux comprend en outre une protéine membranaire extracellulaire, de préférence dans lequel la protéine membranaire extracellulaire comprend du collagène, de la fibronectine ou de la laminine ;
(B) le milieu aqueux comprend en outre un milieu de culture, de préférence dans lequel le milieu de culture est une solution aqueuse d'un ou de plusieurs acides aminés, d'un ou de plusieurs sels, de glucose, et d'une ou de plusieurs vitamines ; et/ou
(C) le milieu aqueux comprend :

(a) un hydrogel à une concentration de 0,5 à 30,0 mg/mL ;
(b) un milieu de culture à une concentration de 60,0 à 90,0 % en volume ; et
(c) un composé comprenant un dipeptide à une concentration de 0,1 à 10,0 mmol/L ; et optionnellement
(d) une protéine membranaire extracellulaire à une concentration de 0,001 à 1,0 mg/mL.

**4.** Procédé selon l'une quelconque des revendications précédentes, dans lequel l'une ou les plusieurs cellules biologiques :

(A) sont sélectionnées parmi deux, ou plus, différents types de cellules biologiques, optionnellement dans lequel

(a) une ou plusieurs des gouttelettes comprennent chacune deux, ou plus, cellules biologiques disposées dans le milieu aqueux, et où les deux, ou plus, cellules biologiques sont sélectionnées parmi deux, ou plus, différents types de cellules biologiques, ou
(b) l'ensemble de gouttelettes comprend

une première pluralité de gouttelettes, chacune desquelles gouttelettes comprend une ou plusieurs d'un premier type de cellules biologiques disposées dans le milieu aqueux, et
une seconde pluralité de gouttelettes, chacune desquelles gouttelettes comprend une ou plusieurs d'un second type de cellules biologiques disposées dans le milieu aqueux ;

(B) sont des cellules mammaliennes ; et/ou
(C) sont disposées dans le milieu aqueux à une concentration de 105 à 109 cellules par mL de milieu aqueux.

**5.** Procédé selon l'une quelconque des revendications précédentes, dans lequel le milieu hydrophobe en masse comprend un composé hydrocarbure et/ou une huile de silicone, de préférence dans lequel le composé hydrocarbure est un alcane en $C_8$ à $C_{16}$, de façon davantage préférée dans lequel le composé hydrocarbure est un undécane ;

optionnellement dans lequel le milieu hydrophobe en masse comprend un mélange d'un hydrocarbure et d'une huile de silicone en un rapport (hydrocarbure) : (huile de silicone) de 50 : 50 à 80 : 20 en volume, de préférence en un rapport de 60 : 40 à 70 : 30 en volume ; et
optionnellement dans lequel l'un ou les plusieurs composés amphipathiques sont sélectionnés parmi des lipides, de préférence dans lequel l'un ou les plusieurs composés amphipathiques sont sélectionnés parmi des phospholipides, de façon davantage préférée dans lequel l'un ou les plusieurs composés amphipathiques sont sélectionnés parmi des lipides phosphocholine.

**6.** Procédé selon l'une quelconque des revendications précédentes, dans lequel la génération de ladite pluralité de gouttelettes comprend :

(i) une pluralité d'étapes de distribution, chacune desquelles étapes de distribution comprend l'injection, dans le milieu hydrophobe en masse, d'un volume du milieu aqueux avec l'une ou les plusieurs cellules biologiques disposées dans celui-ci, optionnellement dans lequel le volume du milieu aqueux avec l'une ou les plusieurs cellules biologiques disposées dans celui-ci est de 0,001 à 100 nL, et optionnellement dans lequel la génération ladite pluralité de gouttelettes comprend 100 ou plus des étapes de distribution ;
et/ou
(ii) l'utilisation d'un appareil pour la génération de gouttelettes,

lequel appareil comprend

(a) au moins un générateur de gouttelette ;
(b) un récipient qui est mobile relativement à l'au moins un générateur de gouttelette ; et
(c) une unité de commande qui est adaptée pour commander la distribution de gouttelettes provenant de l'au moins un générateur de gouttelette et pour commander le mouvement du récipient relativement à l'au moins un générateur de gouttelette,

optionnellement dans lequel :

le ou chaque générateur de gouttelette comprend :

une chambre pour contenir le milieu aqueux avec l'une ou les plusieurs cellules biologiques disposées dans celui-ci ;
une sortie ; et
un composant pour déplacer, à travers ladite sortie, un volume du milieu aqueux avec l'une ou les plusieurs cellules biologiques disposées celui-ci et ainsi distribuer ledit volume sous forme de gouttelette, optionnellement dans lequel
le composant est un transducteur piézoélectrique ; et/ou

le récipient qui est mobile relativement à l'au moins un générateur de gouttelette comprend le milieu hydrophobe en masse.

**7.** Procédé selon l'une quelconque des revendications précédentes, dans lequel au moins une des gouttelettes comprend en outre une couche extérieure de molécules amphipathiques autour de la surface du milieu aqueux, et de préférence entre en contact avec une autre desdites gouttelettes qui comprend en outre une couche extérieure de molécules amphipathiques autour de la surface du milieu aqueux dans lequel une couche de molécules amphipathiques est formée entre les première et seconde gouttelettes en tant qu'interface, optionnellement dans lequel :

(A) l'ensemble de gouttelettes comprend une pluralité de gouttelettes agencées en une structure tridimensionnelle, dans lequel chaque gouttelette dans la structure tridimensionnelle comprend une couche extérieure de molécules amphipathiques autour de la surface du milieu aqueux et dans lequel chaque gouttelette dans la structure tridimensionnelle entre en contact avec au moins une autre gouttelette dans la structure tridimensionnelle, formant une couche de molécules amphipathiques en tant qu'interface entre des gouttelettes entrant en contact ;
(B) la couche de molécules amphipathiques formée entre les première et seconde gouttelettes en tant qu'interface est une bicouche de molécules amphipathiques formée entre les première et seconde gouttelettes en tant qu'interface ; et/ou
(C) la couche extérieure de molécules amphipathiques autour de la surface du milieu aqueux dans une ou plusieurs des gouttelettes comprend en outre une ou plusieurs protéines transmembranaires.

**8.** Procédé selon l'une quelconque des revendications précédentes, lequel procédé comprend en outre :

(A) avant l'étape de la génération d'une pluralité de gouttelettes, une étape de la préparation du milieu aqueux avec une ou plusieurs cellules biologiques disposées dans celui-ci, laquelle étape de préparation comprend la fourniture d'un milieu aqueux et la suspension d'une pluralité de cellules biologiques dans le milieu aqueux ;
(B) une étape de gélification, laquelle étape de gélification comprend la permission au milieu aqueux comprenant le composé hydrogel de former un gel, de préférence dans lequel l'étape de gélification comprend le refroidissement de l'ensemble de gouttelettes jusqu'à une température inférieure ou égale à 10,0 °C et la permission au milieu aqueux comprenant le composé hydrogel de former un gel ;
(C) la mise en culture de la pluralité de cellules pour produire un ensemble de gouttelettes cellularisé ; et/ou
(D) l'isolement de l'ensemble de gouttelettes par rapport au milieu hydrophobe en masse.

**9.** Ensemble de gouttelettes comprenant une pluralité de gouttelettes, dans lequel chacun desdites gouttelettes comprend :

(i) un milieu aqueux comprenant (i) un composé hydrogel, lequel composé hydrogel est un polysaccharide, et (ii) un ou plusieurs agents stabilisants, lequel un ou lesquels plusieurs agents stabilisants sont sélectionné parmi des composés qui comprennent un dipeptide ;
(ii) une ou plusieurs cellules biologiques disposées dans le milieu aqueux ; et
(iii) une couche extérieure de molécules amphipathiques autour de la surface du milieu aqueux,

dans lequel au moins une gouttelette dans l'ensemble de gouttelettes entre en contact avec au moins une autre gouttelette dans l'ensemble de gouttelettes, formant une couche de molécules amphipathiques en tant qu'interface entre des gouttelettes entrant en contact.

**10.** Ensemble de gouttelettes selon la revendication 9, dans lequel le composé hydrogel est de l'agarose.

**11.** Ensemble de gouttelettes selon la revendication 9 ou la revendication 10 dans lequel l'un ou les plusieurs agents stabilisants sont sélectionnés parmi des dipeptides protégés avec un groupe FMOC, de préférence dans lequel l'un

ou les plusieurs agents stabilisants sont FMOC-isoleucine-glycine et/ou FMOC-phénylalanine-phénylalanine.

**12.** Ensemble de gouttelettes selon l'une quelconque des revendications 9 à 11 dans lequel le milieu aqueux comprend une protéine membranaire extracellulaire, de préférence dans lequel la protéine membranaire extracellulaire comprend du collagène, de la fibronectine ou de la laminine.

**13.** Ensemble de gouttelettes selon l'une quelconque des revendications 9 à 12, dans lequel :

(A) l'ensemble de gouttelettes comprend :

une première pluralité de gouttelettes, chacune desquelles gouttelettes comprend une ou plusieurs d'un premier type de cellules biologiques disposées dans le milieu aqueux, et
une seconde pluralité de gouttelettes, chacune desquelles gouttelettes comprend une ou plusieurs d'un second type de cellules biologiques disposées dans le milieu aqueux ;

(B) chacune de la pluralité de gouttelettes a un volume de 0,001 à 100 nL ;
(C) l'ensemble de gouttelettes est tel que défini en outre dans la revendication 3 ; et/ou
(D) le milieu aqueux comprenant un composé hydrogel est sous la forme d'un gel.

**14.** Composition comprenant un milieu hydrophobe en masse et, disposé dans le milieu hydrophobe en masse, un ensemble de gouttelettes tel que défini dans l'une quelconque des revendications 9 à 13.

**15.** Ensemble de gouttelettes pouvant être obtenu par un procédé tel que défini dans l'une quelconque des revendications 1 à 8.

Fig. 1a

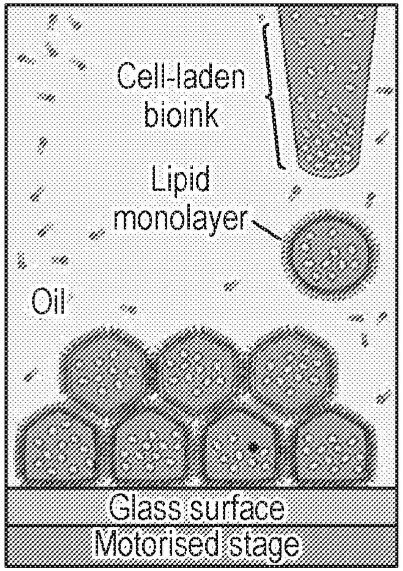


Fig. 1b

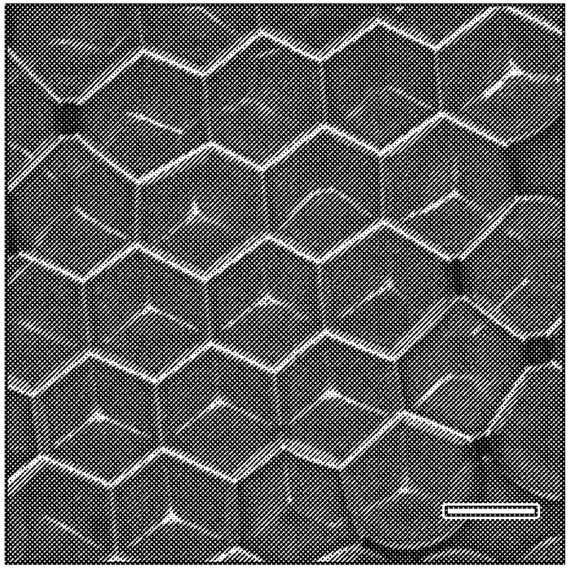

Fig. 1c

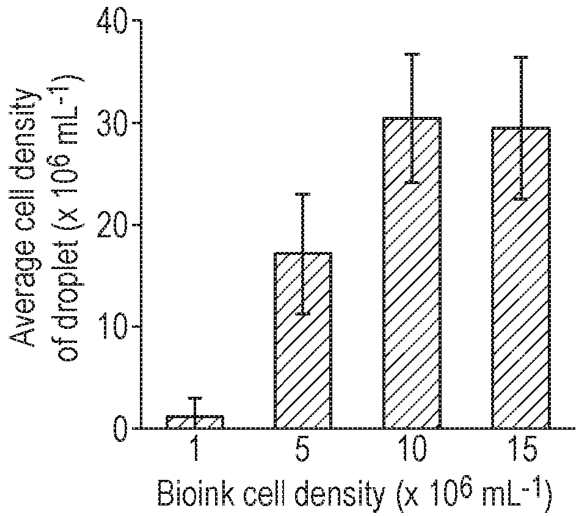

Fig. 1d

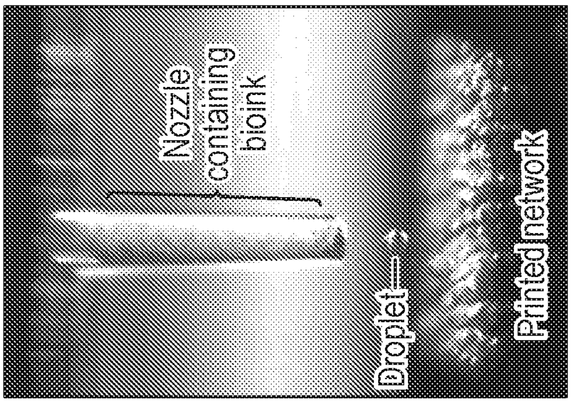


Fig. 1e

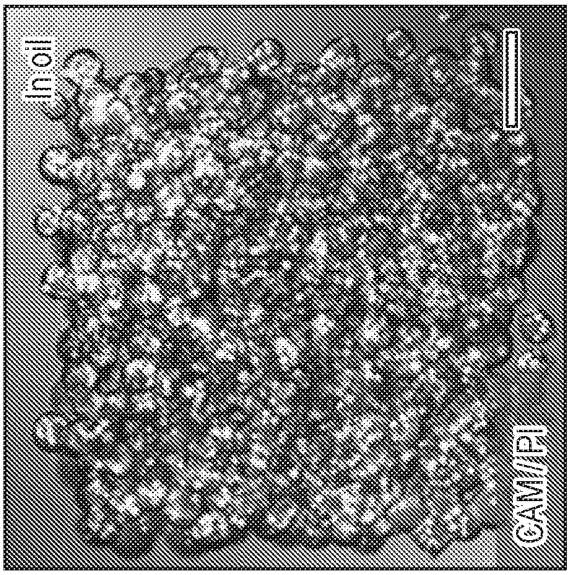


Fig. 1f

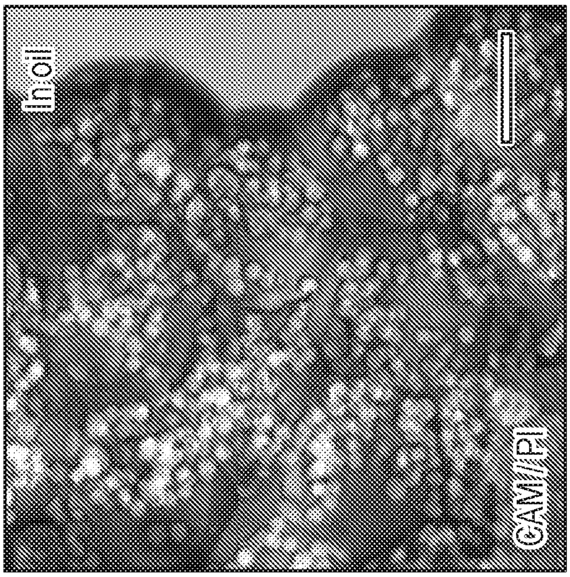

Fig. 2a

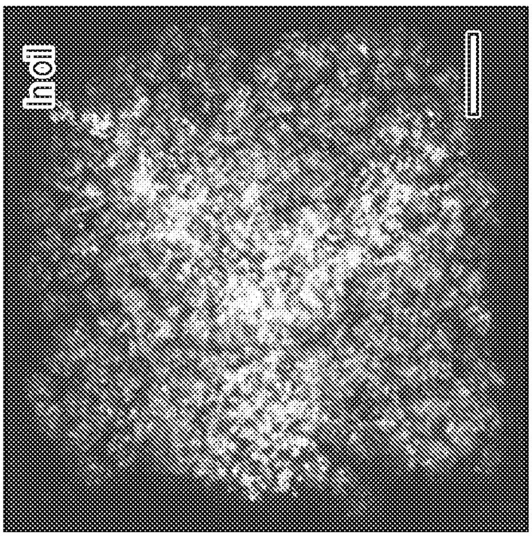


Fig. 2b

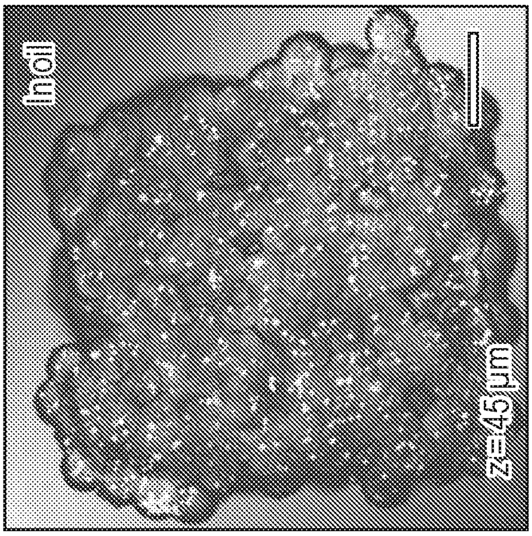


Fig. 2c

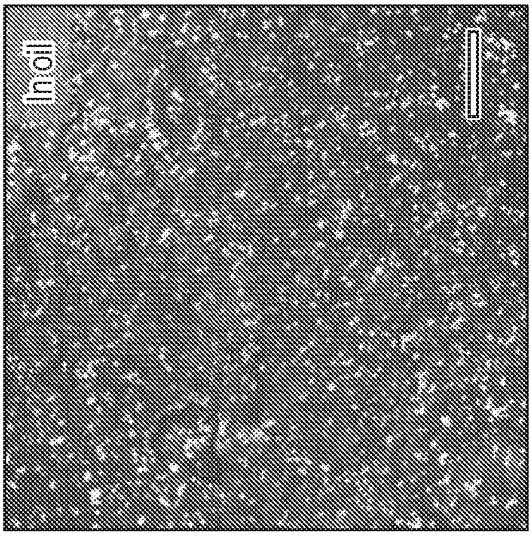

Fig. 2d

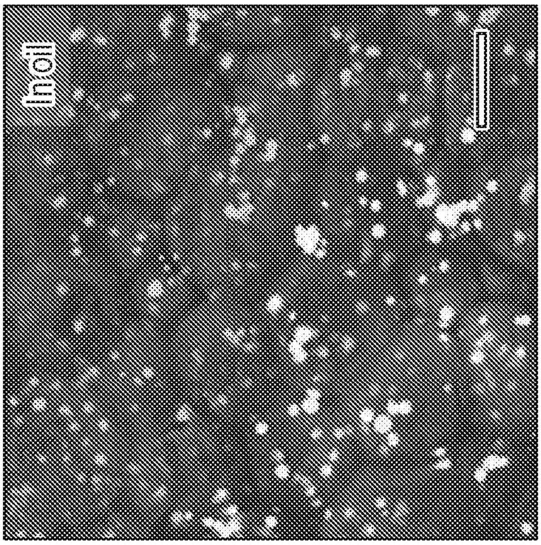


Fig. 2e

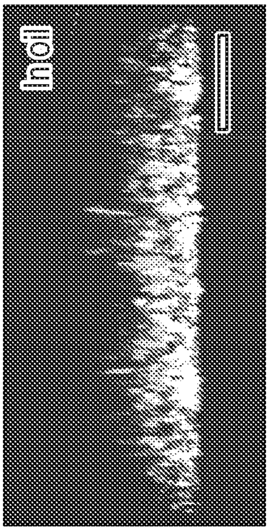


Fig. 2f

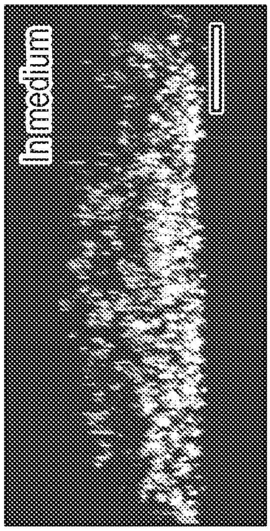


Fig. 2g

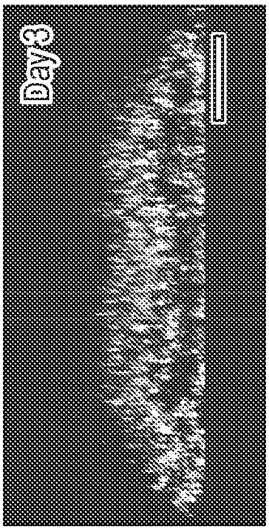


Fig. 2h

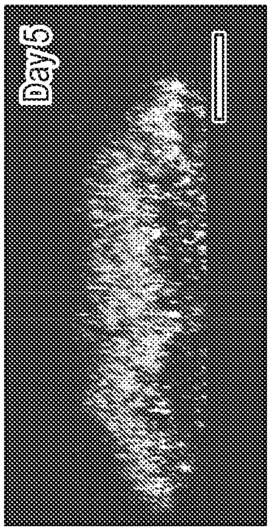

Fig. 3a

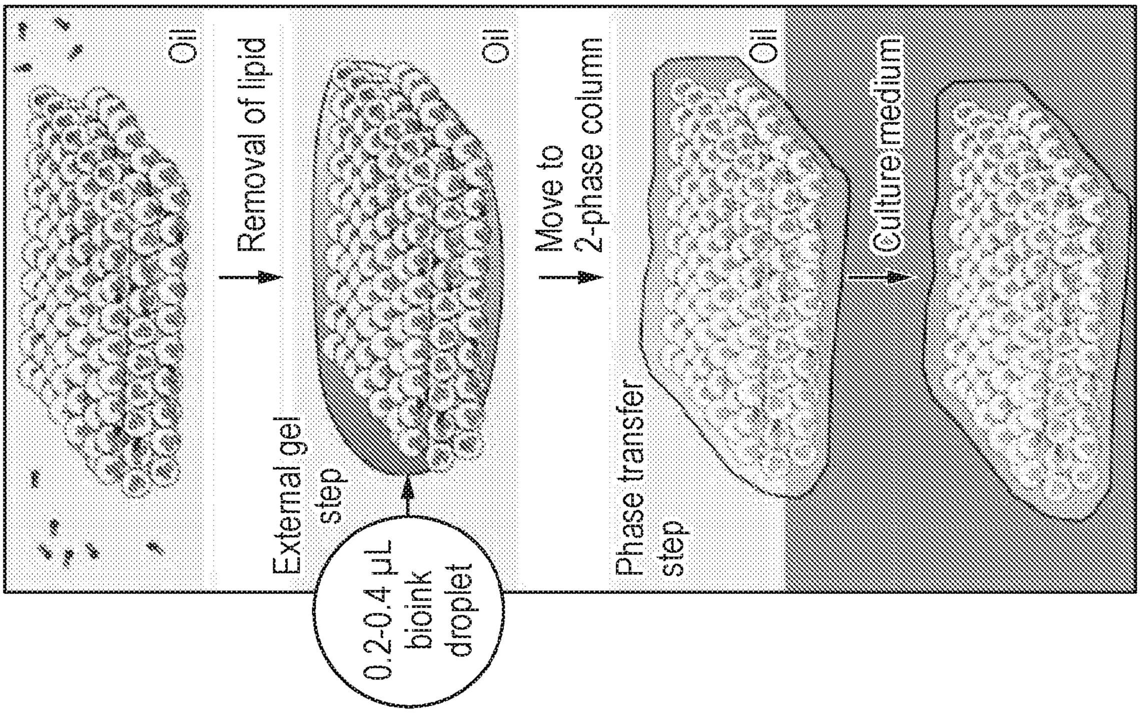
Oil
Removal of lipid
External gel step
0.2-0.4 µL bioink droplet
Oil
Move to 2-phase column
Phase transfer step
Oil
Culture medium

Fig. 3b

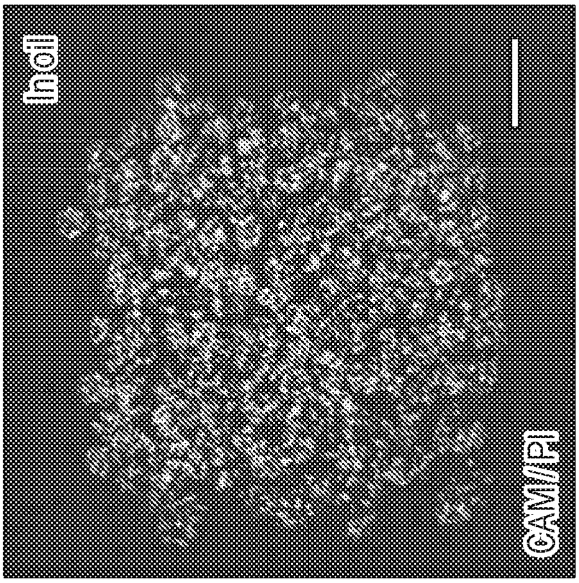
Indil
CAM/PI

Fig. 3c

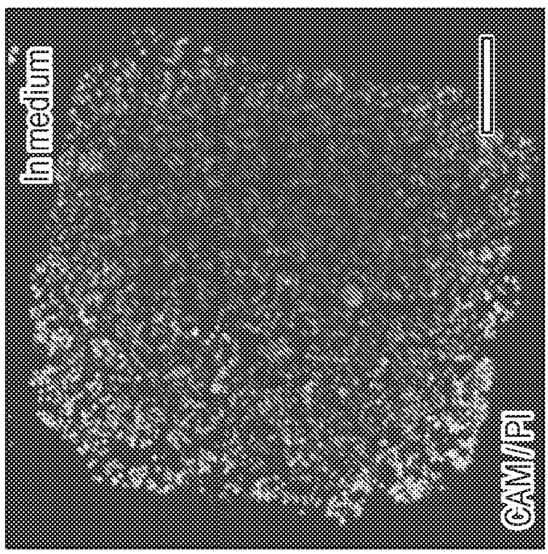


Fig. 3d

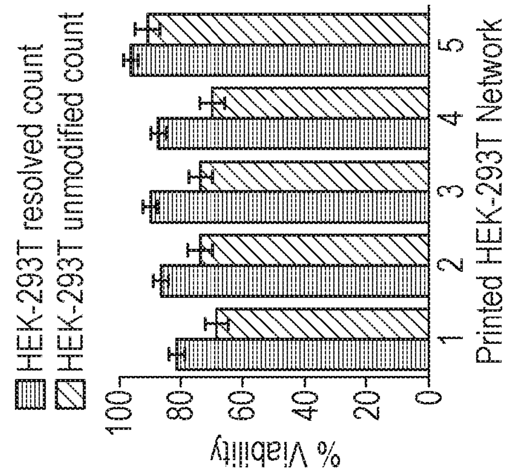


Fig. 3e

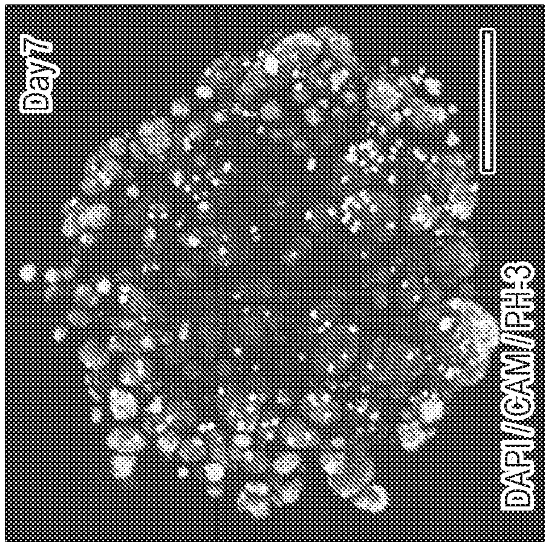

Fig. 4a

Day 0

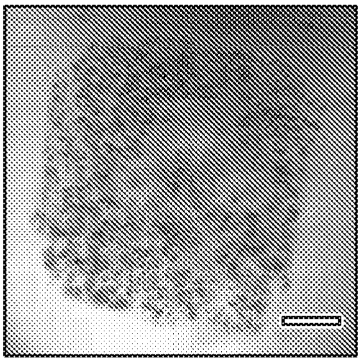

Fig. 4b

Day 3

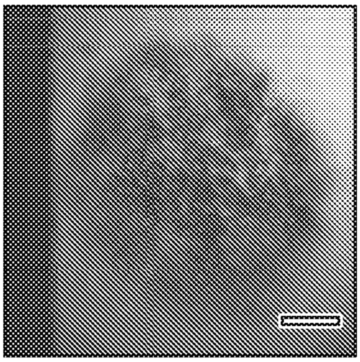

Fig. 4c

Day 7

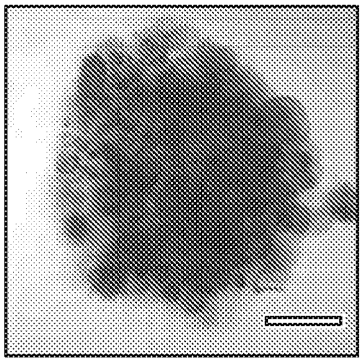

Fig. 4d

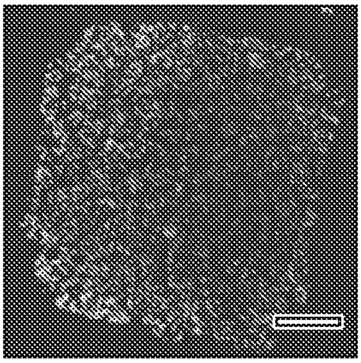

Fig. 4e

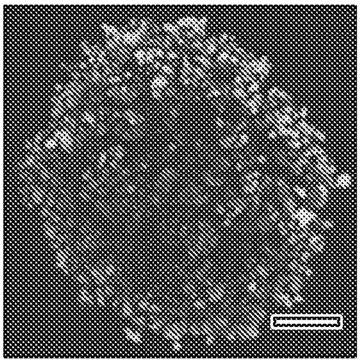

Fig. 4f

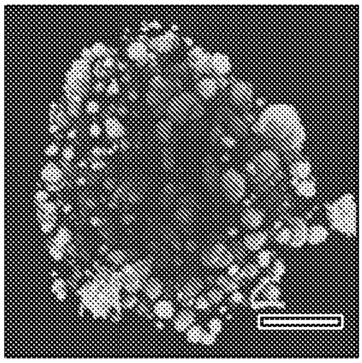

Fig. 4g

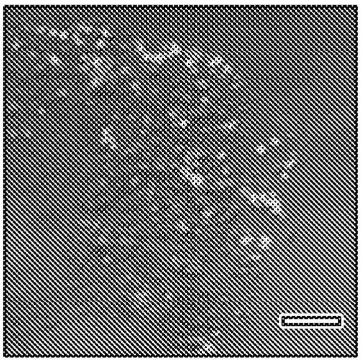

Fig. 4h

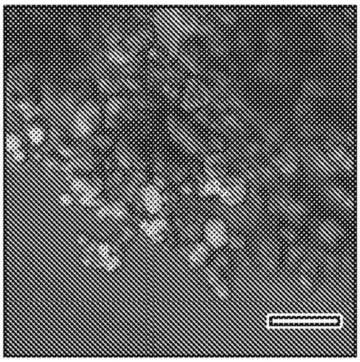

Fig. 4i

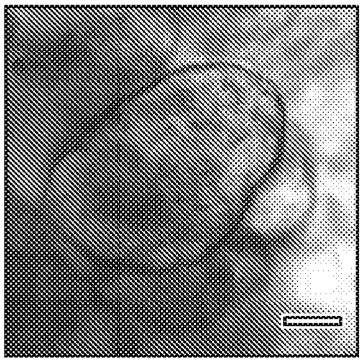

Fig. 5a

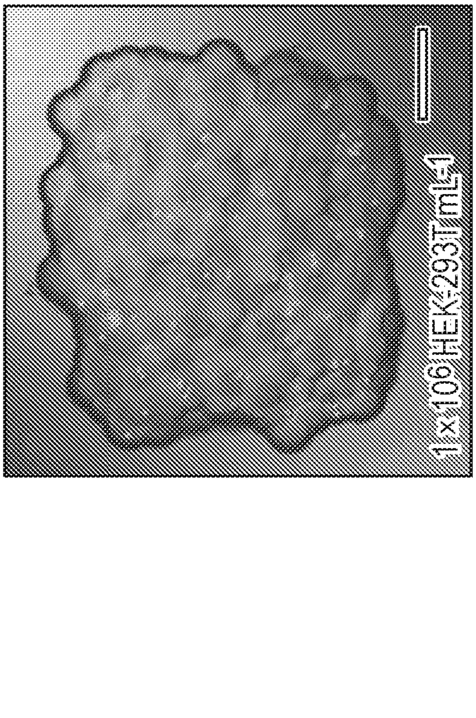


Fig. 5b

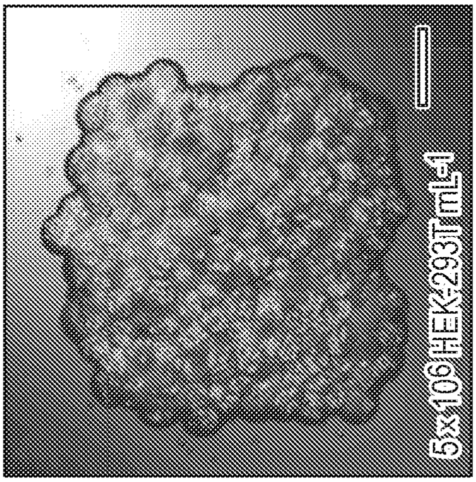


Fig. 5e

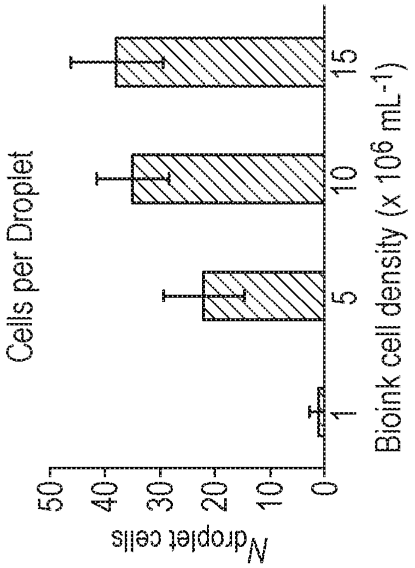


Fig. 5c

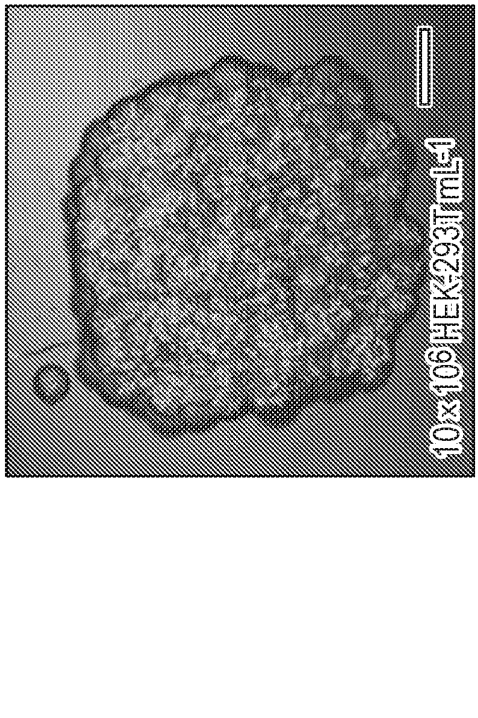


Fig. 5d

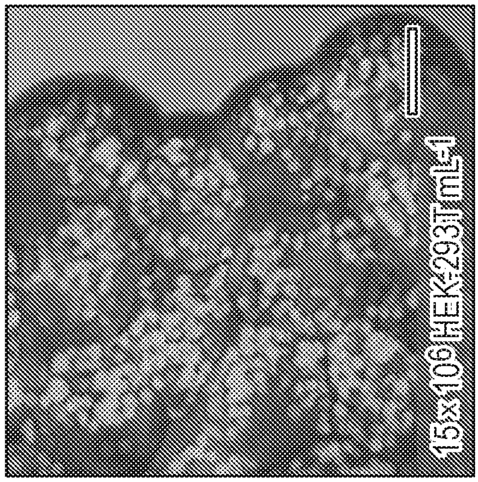


Fig. 5f

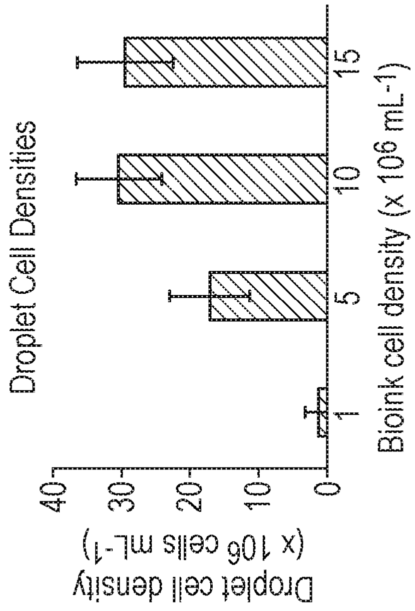

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014064459 A
- WO 2014087175 A


### Non-patent literature cited in the description


- **SYEDA et al.** *J. Am. Chem. Soc.,* 2008, vol. 130, 15543-8
- **VILLAR et al.** *Science,* 2013, vol. 340, 48-52
- **MAGLIA et al.** *Nat. Nanotechnol.,* 2009, vol. 4, 437-440
- **ELANI et al.** *Lab Chip,* 2012, vol. 12, 3514-20
- **VILLAR et al.** *Nat. Nanotechnol.,* 2011, vol. 6, 803-8
- **ELANI et al.** *Chem. Sci.,* 2013, vol. 4, 3332
- **ONOE et al.** *Drug Discov. Today,* 2015, vol. 20, 236-246
- **LANCASTER et al.** *Science,* 2014, vol. 345 (80), 1247125
- **FOTY, R.** *J. Vis. Exp.,* 2011, vol. 20, 4-7
- **HARAGUCHI, Y.** *RSC Adv.,* 2012, vol. 2, 2184
- **ONOE, H.** *Drug Discov. Today,* 2015, vol. 20, 236-246
- **ONOE, H.** *Nat. Mater.,* 2013, vol. 12, 584-90
- **LI et al.** *Angew. Chemie Int. Ed.,* 2015, vol. 54, 1-6
- **GURKAN et al.** *Mol. Pharm.,* 2014, vol. 11, 2151-9
- **DERBY.** *Science,* 2012, vol. 338, 921-6
- **KOLESKY et al.** *Adv. Mater.,* 2014, vol. 26, 3124-30